# EUROPEAN PATENT APPLICATION

(11) **EP 2 338 890 A1**
(43) Date of publication of application: **29.06.2011**
(21) Application number: 09180371.8
(22) Date of filing: 22.12.2009
(51) Int. Cl.: C07D 487/04, A01N 43/60

(54) **4,7-Diazaindole derivatives and their use as fungicides**

(71) Applicant: Bayer CropScience AG, 40789 Monheim (DE)
(72) Inventor: Mattes, Amos, 40674 Langenfeld (DE); Hillebrand, Stefan, 41462 Neuss (DE); Helmke, Hendrik, 65835 Liederbach (DE); Sudau, Alexander, 42799 Leichlingen (DE); Benting, Jürgen, 42799 Leichlingen (DE); Dahmen, Peter, 41470 Neuss (DE); Wachendorff-Neumann, Ulrike, 56566 Neuwied (DE); Braun, Christoph Andreas, 40625 Düsseldorf (DE); Meissner, Ruth, 51375 Leverkusen (DE)

(57) **Abstract**

The present invention relates to compounds of formula (I) 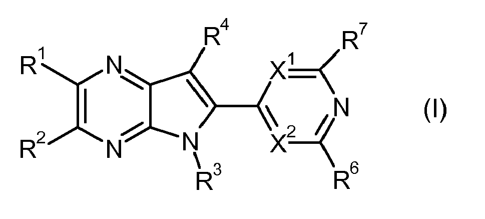 their process of preparation, their use for preventing and/or controlling fungal infection in plants and/or plant propagation material and for reducing mycotoxin contamination in plants or plant material, and compositions containing these compounds.

## Description

The present invention relates to novel fungicidally active and mycotoxin-reducing 4,7-diaza-indoles, compositions comprising these novel compounds and their use in methods for the control and/or prevention of fungal infection in plants. In addition, the invention relates to processes for preparing the novel compounds of the invention.

Certain 4,7-diaza-indoles and their use in the prevention and treatment of human and animal disease, although not that caused by fungi, are described in WO-A 1999/20624. Similar compounds are described in WO-A 2001/047922, WO-A 2003/000688.

It has now been found that certain 4,7-diaza-indoles have fungicidal and mycotoxin-reducing activity and, in particular, activity against plant pathogenic and mycotoxin-producing fungi and can be used for the reduction of mycotoxin contamination in plants or plant material.

Accordingly, the present invention provides 4,7-diaza-indoles as compounds of formula (I) 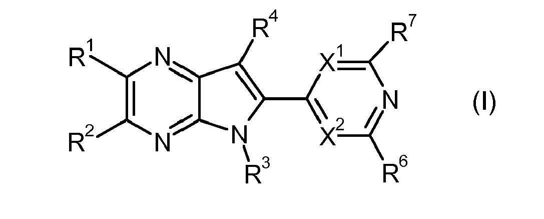 wherein:
X¹ is N or CH;
X₂ is N or CR⁵
R¹ and R² are independently
   (i) hydrogen, halogen, hydroxyl, cyano or nitro,
   (ii) optionally substituted alkyl, alkenyl or alkynyl,
   (iii) optionally substituted aryl, heteroaryl, cyclyl or heterocyclyl, or
   (iv) C(O)R¹⁰, -C(O)NR¹⁰R¹¹, -C(S)NR¹⁰R¹¹, -C(NOR¹⁰)Rⁿ, -C(O)OR¹⁰, -OR¹⁰, - SR¹⁰,-S(O)R¹⁰, -S(O)NR¹⁰R¹¹, -S(O)₂NR¹⁰R¹¹, -S(O)₂R¹⁰, -NR¹⁰R¹¹, -NC(O)R¹⁰, -P(O)(OR¹⁰)(OR¹¹) or-OP(O)(OR¹⁰)(OR¹¹)_{;}
R³ is
   (i) hydrogen, hydroxyl, cyano or nitro,
   (ii) optionally substituted alkyl, alkenyl, allenyl, alkynyl or haloalkyl, (iii) optionally substituted aryl, heteroaryl, cyclyl or heterocyclyl or heteroaralkyl or (iv) -C(O)R¹², -C(O)OR¹², -OR¹², -OC(O)R¹², -S(O)₂R¹² or -NR¹²R¹³;
R⁴ is
   (i) hydrogen, halogen, hydroxyl, cyano or nitro,
   (ii) optionally substituted alkyl, alkenyl, allenyl, alkynyl or haloalkyl,
   (iii) optionally substituted aryl, heteroaryl, cyclyl or heterocyclyl or
   (iv) -C(O)R¹⁴, -C(O)OR¹⁴, -C(NOR¹⁴)R¹⁵, -OR¹⁴, -SR¹⁴, -S(O)NR¹⁴R¹⁵, -S(O)₂R¹⁴,
      or -NR¹⁴R¹⁵;
R⁵ is
   (i) hydrogen, halogen, hydroxyl, cyano or nitro,
   (ii) optionally substituted alkyl, alkenyl or alkynyl,
   (iii) -C(O)R¹⁶, -C(O)OR¹⁶, -OR¹⁶, -SR¹⁶, -S(O)R¹⁶, -S(O)NR¹⁶R¹⁷, -S(O)₂R¹⁶, or -
      NR¹⁶R¹⁷;
R⁶ is hydrogen, halogen, cyano, C(O)OR¹⁸, -SR¹⁸, NR¹⁸R¹⁹, -C(O)NR¹⁸R¹⁹ or -N=CR²⁰,-
   C(=NR¹⁸)NR¹⁹R²⁰ or optionally substituted aryl, heteroaryl, cyclyl or heterocyclyl;
R⁷ is hydrogen, halogen, hydroxyl, cyano, nitro or optionally substituted alkyl;
R¹⁰, R¹¹, R¹⁴, R¹⁵, R¹⁶ and R¹⁷ are, independently, hydrogen, halogen, hydroxyl, cyano, nitro,
   optionally substituted alkyl, alkoxy, alkenyl or alkynyl, or optionally substituted aryl, heteroaryl, cyclyl or heterocyclyl;
R¹² and R¹³ are, independently, hydrogen, halogen, hydroxyl, cyano, nitro, -NR²¹ R²², optionally
   substituted alkyl, alkoxy, alkenyl or alkynyl, or optionally substituted aryl, heteroaryl, cyclyl or heterocyclyl;
R¹⁸ and R¹⁹ are, independently,
   (i) hydrogen,
   (ii) optionally substituted alkyl, alkenyl or alkynyl,
   (iii) optionally substituted aryl, heteroaryl, cyclyl or heterocyclyl, or
   (iv) -C(S)R²³ -C(O)R²³, -SO₂R²³, -C(O)OR²³, -OR²³ or C(O)NR²³R²⁴;
R²⁰ is hydroxyl, optionally substituted alkyl or alkoxy or -NR²¹R²², or -N=CR²¹R²²;
R²¹ and R²² are, independently, hydrogen, optionally substituted alkyl, alkenyl or alkynyl, optionally substituted cyclyl, heterocyclyl, aryl, or heteroaryl or aralkyl or - C(O)OR²⁵;
R²³ and R²⁴ are, independently, hydrogen, hydroxyl, optionally substituted alkyl, alkenyl or alkynyl, or optionally substituted aryl, heteroaryl, cyclyl or heterocyclyl or aralkyl; and
R²⁵ is optionally substituted alkyl, alkenyl or alkynyl;
   and/or
   independently, (i) R¹ and R², (ii) R³ and R⁵, (iii) R⁵ and R⁶, (iv) R⁵ and R¹⁸, (v) R⁵ and R¹⁹, (vi) R¹⁴ and R¹⁵ and (vii) R¹⁸ and R¹⁹ form an optionally substituted aryl, heteroaryl, cyclyl or heterocyclyl group containing from 5 to 18 ring atoms;
   or a salt or a N-oxide thereof
   with the proviso that the compounds from the group consisting of
   N-[4-[7-(4-fluorophenyl)-4H-pyrrolo[2,3-b]pyrazin-6-yl]-2-pyridinyl]-glycine, N1-[4-[7-(4-fluorophenyl)-5H-pyrrolo[2,3-b]pyrazin-6-yl]-2-pyridinyl]-1,2-ethanediamine, 4-[7-(4-fluorophenyl)-5H-pyrrolo[2,3-b]pyrazin-6-yl]-N-methyl-2-pyridinamine, 7-(4-fluorophenyl)-5-methyl-6-(4-pyridinyl)-5H-pyrrolo[2,3-b]pyrazine, 7-(3-chlorophenyl)-6-(2-chloro-4-pyridinyl)-5H-pyrrolo[2,3-b]pyrazine, N-[4-[7-(4-fluorophenyl)-5H-pyrrolo[2,3-b]pyrazin-6-yl]-2-pyridinyl]-1-piperidineacetamide, 7-(4-fluorophenyl)-3-methoxy-6-(4-pyridinyl)- 5H-pyrrolo[2,3-b]pyrazine, 7-(4-fluorophenyl)-N,N-dimethyl-6-(4-pyridinyl)- 5H-pyrrolo[2,3-b]pyrazin-3-amine, 2-[[4-[7-(4-fluorophenyl)-3-(methylamino)-5H-pyrrolo[2,3-b]pyrazin-6-yl]-2-pyridinyl]amino]-ethanol, N-[4-[7-(4-fluorophenyl)-5H-pyrrolo[2,3-b]pyrazin-6-yl]-2-pyridinyl]-4-methyl-1-piperazineacetamide, N-[4-[7-(4-fluorophenyl)-5H-pyrrolo[2,3-b]pyrazin-6-yl]-2-pyridinyl]-1H-imidazole-1-acetamide, 7-(4-fluorophenyl)-N-methyl-6-[2-(methylamino)-4-pyridinyl]- 5H-pyrrolo[2,3-b]pyrazin-3-amine, 4-[7-(3-chlorophenyl)-5H-pyrrolo[2,3-b]pyrazin-6-yl]-N-[2-(methylthio)ethyl]- 2-pyridinamine, 5-ethyl-7-(4-fluorophenyl)-6-(4-pyridinyl)- 5H-pyrrolo[2,3-b]pyrazine, N1-[4-[7-(4-fluorophenyl)-5H-pyrrolo[2,3-b]pyrazin-6-yl]-2-pyridinyl]- 1,3-propanediamine, 7-(4-fluorophenyl)-N-(3-methoxypropyl)-6-[2-[(3-methoxypropyl)amino]-4-pyridinyl]- 5H-pyrrolo[2,3-b]pyrazin-3-amine, 7-(4-fluorophenyl)-N-methyl-6-(4-pyridinyl)- 5H-pyrrolo[2,3-b]pyrazine-5-propanamine, N-[4-[3-(acetylamino)-7-(4-fluorophenyl)-5H-pyrro-1o[2,3-b]pyrazin-6-yl]-2-pyridinyl]-acetamide, 3-chloro-6-(2-chloro-4-pyridinyl)-7-(4-fluorophenyl)- 5H-pyrrolo[2,3-b]pyrazine, 2-[[4-[7-(4-fluorophenyl)-5H-pyrrolo[2,3-b]pyrazin-6-yl]-2-pyridinyl]amino]-ethanol, N-[4-[3-[(3-aminopropyl)amino]-7-(4-fluorophenyl)-4H-pyrro-1o[2,3-b]pyrazin-6-yl]-2-pyridinyl]- 1,3-propanediamine, 7-(4-fluorophenyl)-N-(2-methoxyethyl)-6-[2-[(2-methoxyethyl)amino]-4-pyridinyl]- 5H-pyrrolo[2,3-b]pyrazin-3-amine, 7-(4-fluorophenyl)-3-(methylthio)-6-(4-pyridinyl)- 5H-pyrrolo[2,3-b]pyrazine, 4-[[7-(4-fluorophenyl)-6-[2-[(4-hydroxybutyl)amino]-4-pyridinyl]-4H-pyrrolo[2,3-b]pyrazin-3-yl]amino]- 1-butanol, N-[4-[7-(4-fluorophenyl)-5H-pyrrolo[2,3-b]pyrazin-6-yl]-2-pyridinyl]-2-methoxy-acetamide, 7-(4-fluorophenyl)-5-[3-(4-nitro-1H-imidazol-1-yl)propyl]-6-(4-pyridinyl)-5H-pyrrolo[2,3-b]pyrazine, 2-[[7-(4-fluorophenyl)-6-[2-[(2-hydroxyethyl)amino]-4-pyridinyl]-4H-pyrrolo[2,3-b]pyrazin-3-yl]amino]- ethanol, 2-(dimethylamino)-N-[4-[7-(4-fluorophenyl)-5H-pyrrolo[2,3-b]pyrazin-6-yl]-2-pyridinyl]-acetamide, 3-[[7-(4-fluorophenyl)-6-[2-[[(2-hydroxyethyl)methyl]amino]-4-pyridinyl]-4H-pyrrolo[2,3-b]pyrazin-3-yl]amino]-1-propanol, 7-(4-fluorophenyl)-5-[3-(2-nitro-1H-imidazol-1-yl)propyl]-6-(4-pyridinyl)- 5H-pyrrolo[2,3-b]pyrazine, 3-(dimethylamino)-N-[4-[7-(4-fluorophenyl)-5H-pyrrolo[2,3-b]pyrazin-6-yl]-2-pyridinyl]-propanamide, 7-(4-fluorophenyl)-5-[3-(4-methyl-1-piperazinyl)propyl]-6-(4-pyridinyl)- 5H-pyrrolo[2,3-b]pyrazine, 7-(4-fluorophenyl)-5-[3-(4-morpholinyl)propyl]-6-(4-pyridinyl)- 5H-pyrrolo[2,3-b]pyrazine, N-[4-[7-(4-fluorophenyl)-5H-pyrrolo[2,3-b]pyrazin-6-yl]-2-pyridinyl]-2-(methylamino)-acetamide, N-[7-(4-fluorophenyl)-6-(4-pyridinyl)-5H-pyrrolo[2,3-b]pyrazin-3-yl]-acetamide, 6-(4-pyridinyl)- 5H-pyrrolo[2,3-b]pyrazine, 6-(2-bromo-4-pyridinyl)-7-(4-fluorophenyl)-5H-pyrrolo[2,3-b]pyrazine, 7-(4-fluorophenyl)-5-[3-(4-methyl-1H-imidazol-1-yl)propyl]-6-(4-pyridinyl)- 5H-pyrrolo[2,3-b]pyrazine, 5-(3-chloropropyl)-7-(4-fluorophenyl)-6-(4-pyridinyl)-5H-pyrrolo[2,3-b]pyrazine, 7-(4-fluorophenyl)-6-(4-pyridinyl)-5H-pyrrolo[2,3-b]pyrazine, N-[4-[7-(4-fluorophenyl)-5H-pyrrolo[2,3-b]pyrazin-6-yl]-2-pyridinyl]-acetamide, 4-[7-(4-fluorophenyl)-5H-pyrrolo[2,3-b]pyrazin-6-yl]-2-pyridinamine, 6-(2-chloro-4-pyridinyl)-7-(4-fluorophenyl)-5H-pyrrolo[2,3-b]pyrazine, 3-[[4-[7-(4-fluorophenyl)-5H-pyrrolo[2,3-b]pyrazin-6-yl]-2-pyridinyl]amino]-1-propanol, 7-(4-fluorophenyl)-5-[3-(1H-imidazol-1-yl)propyl]-6-(4-pyridinyl)-5H-pyrrolo[2,3-b]pyrazine, 7-(4-fluorophenyl)-6-(4-pyridinyl)-5H-pyrrolo[2,3-b]pyrazine-5-butanenitrile are excluded.

The compounds of formula (I) as described above are suitable for preventing and/or controlling fungal infection in plants and/or plant propagation material and for reducing mycotoxin contamination in plants or plant material.

In a particular embodiment the compounds of formula (I) as described above are suitable for preventing and/or controlling fungal infection in plants and/or plant propagation material.

In a particular embodiment the compounds of formula (I) as described above are suitable for preventing and controlling fungal infection in plants and/or plant propagation material.

In a particular embodiment the compounds of formula (I) as described above are suitable for preventing or controlling fungal infection in plants and/or plant propagation material.

In a particular embodiment the compounds of formula (I) as described above are suitable for reducing mycotoxin contamination in plants and/or plant material.

Unless otherwise stated, the following terms used in the specification and claims have the meanings given below:
**"Alkyl"** means a linear saturated monovalent hydrocarbon radical of one to eight carbon atoms or a branched saturated monovalent hydrocarbon radical of three to eight carbon atoms, e.g. methyl, ethyl, *n*-propyl, *iso*-propyl, *n*-butyl, *sec*-butyl, *iso*-butyl, *tert*-butyl, *n*-pentyl, *n*-hexyl and the like. Preferably, linear alkyl groups contain one to six carbon atoms, more preferably one to four carbon atoms and most preferably are selected from methyl, ethyl or *n*-propyl. Preferably, branched alkyl groups contain three to six carbon atoms and more preferably are selected from iso-propyl, *sec*-butyl, *iso*-butyl or *tert-* butyl.
**"Alkenyl"** means a linear monovalent saturated hydrocarbon radical of two to eight carbon atoms, or a branched monovalent hydrocarbon radical of three to eight carbon atoms containing at least one double bond, e.g. ethenyl, propenyl and the like. Where appropriate, an alkenyl group can be of either the (*E*)- or (*Z*)-configuration. Preferably, linear alkenyl groups contain two to six carbon atoms and more preferably are selected from ethenyl, prop- 1-enyl, prop-2-enyl, prop-1,2-dienyl, but-1-enyl, but-2-enyl, but-3-enyl, but-1,2-dienyl and but-1,3-dienyl. Preferably, branched alkenyl groups contain three to six carbon atoms and more preferably are selected from 1-methylethenyl, 1-methylprop-1-enyl, 1-methylprop-2- enyl, 2-methylprop-1-enyl and 2-methylprop-2-enyl.
**"Allenyl"** means a linear monovalent saturated hydrocarbon radical of three to eight carbon atoms, or a branched monovalent hydrocarbon radical of three to eight carbon atoms containing at least two double bonds between three contiguous carbon atoms, e.g. propa-1,2 dienyl, penta-1,2 dienyl, penta-2,3 dienyl, hexa-1,2-dienyl and the like. Where appropriate, an alkenyl group can be of either the (*R*)- or (*S*)-configuration. Preferred is propa-1,2- dienyl.
**"Alkynyl"** means a linear monovalent saturated hydrocarbon radical of two to eight carbon atoms, or a branched monovalent hydrocarbon radical of four to eight carbon atoms, containing at least one triple bond, e.g. ethynyl, propynyl and the like. Preferably, linear alkynyl groups contain two to six carbon atoms and more preferably are selected from ethynyl, prop-1-ynyl, prop-2-ynyl, but-1-ynyl, but-2-ynyl and but-3-ynyl. Preferably, branched alkynyl groups contain four to six carbon atoms and more preferably are selected from 1-methylprop-2-ynyl, 3-methylbut-1-ynyl, 1-methylbut-2-ynyl, 1-methylbut-3-ynyl and 1 -methylbut-3-ynyl.
**"Alkylene"** means a linear saturated divalent hydrocarbon radical of one to six carbon atoms or a branched saturated divalent hydrocarbon radical or three to six carbon atoms, e.g. methylene, ethylene, propylene, 2-methylpropylene and the like. Preferred alkylene groups are the divalent radicals of the alkyl groups defined above.
**"Alkenylene"** means a linear divalent hydrocarbon radical of two to six carbon atoms or a branched divalent hydrocarbon radical of three to six carbon atoms, containing at least one double bond, e.g. ethenylene, propenylene and the like. Preferred alkenylene groups are the divalent radicals of the alkenyl groups defined above.
"Cyclyl" means a monovalent cyclic hydrocarbon radical of three to eight ring carbons, preferably three to six ring carbons, e.g. cyclopropyl, cyclohexyl and the like.Cyclyl groups may be fully saturated or mono- or di-unsaturated. Preferably, saturated cyclyl groups contain three to six ring carbons, more preferably they are selected from cyclopropyl, cyclobutyl, cyclopentyl and cyclohexyl. Mono-unsaturated cyclyl groups are preferably selected from cyclobutenyl, cyclopentenyl and cyclohexenyl.
**"Heterocyclyl"** means a cyclyl radical containing one, two or three ring heteroatoms selected from N, O or S(O)ₙ (where n is an integer from 0 to 2), the remaining ring atoms being carbon where one or two carbon atoms may optionally be replaced by a carbonyl group. Examples of such rings include, but are not limited to, oxirane, oxetane, tetrahydrofuran, tetrahydropyran, 1,3-dioxolane, 1,4-dioxane, aziridine, azetidine, pyrrolidine, piperidine, oxazinane, morpholine, thiomorpholine, imidazolidine, pyrazolidine and piperazine. More preferably, the heterocyclyl group contains three to five ring atoms including one O and/or one N ring atom.
**"Aryl"** means a monovalent moncyclic or bicyclic aromatic hydrocarbon radical of six to ten ring carbons atoms. Suitable aryl groups include phenyl and naphthyl, in particular, phenyl.
**"Heteroaryl"** means a monovalent monocyclic or bicyclic aromatic hydrocarbon radical of five to ten ring atoms, preferably five or six ring atoms, containing one, two, three or four ring heteroatoms selected, independently, from N, O or S, the remaining ring atoms being carbon. Examples of heteroaryl groups include, but are not limited to pyridyl, pyrimidinyl, pyrazolyl, thiazolyl, thiophenyl, isoazolyl, and tetrazolyl groups.
**"Alkoxy"** means a radical-OR, where R is optionally substituted alkyl, alkenyl or alkynyl or an optionally substituted cyclyl, heterocyclyl, aryl or heteroaryl group or an aralkyl or heteroaralkyl group. Preferably, alkoxy groups are selected from methoxy, ethoxy, 1 -methyl ethoxy, propoxy, 1-methylpropoxy and 2-methylpropoxy. More preferably alkoxy means methoxy or ethoxy.
**"Halo"** or **"halogen"** means fluoro, chloro, bromo or iodo, preferably chloro or fluoro.
**"Haloalkyl"** means alkyl as defined above substituted with one or more of the same or different halo atoms. Examples of haloalkyl groups include, but are not limited to fluoromethyl, difluoromethyl, trifluoromethyl, 2-fluoroethyl, 2-trifluoroethyl, 2-chloro-ethyl, 2-iodoethyl, 3-fluoropropyl, 3-chloropropyl, 2-trifluoro-1-chloroethyl and 1 -difluoro-2- difluoro-3-trifluoropropyl.
**"Haloalkenyl"** means alkenyl as defined above substituted with one or more of the same or different halo atoms. Examples of haloalkenyl groups include, but are not limited to 2-dibromoethenyl, 2-fluoro-2-bromoethenyl, 5-bromopent-3-enyl and 3 dichloroprop-2-enyl.
**"Aralkyl"** means a radical -R^{a}R^{b} where R^{a} is an alkylene or alkenylene group and R^{b} is an aryl group as defined above.
**"Heteroaralkyl"** means a radical -R^{a}R^{b} where R^{a} is an alkylene or alkenylene group and R^{b} is a heteroaryl group as defined above.
**"Acyl"** means -C(O)R, wherein R is hydrogen, optionally substituted alkyl, alkenyl or alkynyl or optionally substituted cyclyl, heterocyclyl, aryl or heteroaryl.
**"Acyloxy"** means a radical -OC(O)R where R is hydrogen, optionally substituted alkyl, alkenyl or alkynyl or optionally substituted cyclyl, heterocyclyl, aryl or heteroaryl.

The groups defined above, in particular, alkyl, alkenyl, alkynyl, cyclyl, heterocyclyl, aryl and heteroaryl groups, may be optionally substituted by one or more substituents independently selected from halogen, hydroxyl, cyano, alkyl (optionally substituted by cyano), haloalkyl, alkenyl, haloalkenyl, alkynyl (optionally substituted by -C(O)OR), haloalkynyl, cyclyl (optionally substituted by cyano, halogen, hydroxyl or methyl), heterocyclyl, aryl (optionally substituted by halogen), heteroaryl, alkoxy (optionally substituted by alkoxy or acyl), -C(O)R, - C(O)OR, -SR, -S(O)R, -S(O)₂R, -S(O)NRR', -OS(O)NRR', -P(O)(OR)(OR'), - 0(P)(O)(OR)(OR'), -NRR', -NHC(O)OR', -C(O)NRR', -O-N=CRR' or trialkylsilyl, wherein R and R' are, independently, hydrogen or alkyl, alkoxy, haloalkyl, alkenyl, haloalkenyl, alkynyl, cyclyl, heterocyclyl, aryl or heteroaryl. In particular, R and R' are, independently, hydrogen or alkyl (in particular, methyl or ethyl). Preferred optional substituents are alkoxy (in particular, methoxy or ethoxy), hydroxyl, cyano, halogen (in particular, fluoro, chloro or bromo), heterocyclyl (in particular, oxirane or tetrahydrofuran), heteroaryl (in particular, pyridyl), - C(O)OR (wherein R is hydrogen or alkyl (in particular, methyl or ethyl)) and trialkylsilyl (in particular, trimethylsilyl).

The compounds of formula (I) may exist in different geometric or optical isomeric forms or in different tautomeric forms. One or more centres of chirality may be present, in which case compounds of the formula (I) may be present as pure enantiomers, mixtures of enantiomers, pure diastereomers or mixtures of diastereomers. There may be double bonds present in the molecule, such as C=C or C=N bonds, in which case compounds of formula (I) may exist as single isomers of mixtures of isomers. Centres of tautomerisation may be present. This invention covers all such isomers and tautomers and mixtures thereof in all proportions as well as isotopic forms such as deuterated compounds.

Suitable salts of the compounds of formula (I) include acid addition salts such as those with an inorganic acid such as hydrochloric, hydrobromic, sulfuric, nitric or phosphoric acid, or an organic carboxylic acid such as oxalic, tartaric, lactic, butyric, toluic, hexanoic or phthalic acid, or a sulphonic acid such as methane, benzene or toluene sulphonic acid. Other examples of organic carboxylic acids include haloacids such as trifiuoroacetic acid.

N-oxides are oxidised forms of tertiary amines or oxidised forms of nitrogen containing heteroaromatic compounds. They are described in many books for example in "Heterocyclic N-oxides" by Angelo Albini and Silvio Pietra, CRC Press, Boca Raton, Florida, 1991.

In particularly preferred embodiments of the invention, the preferred groups for X¹ and X² and R¹ to R²⁵, in any combination thereof, are as set out below. Preferably, X¹ is CH.

Preferably, X² is CR⁵. More preferably, X² is CH.
Preferably, X¹ and X² are both CH.

Preferably, R¹ is hydrogen, halogen or C₁₋₆ alkyl. Preferably, R¹ is hydrogen, halogen or C₁₋₄ alkyl. More preferably, R¹ is hydrogen, chloro or methyl. Most preferably, R¹ is hydrogen.

Preferably, R² is hydrogen, halogen, cyano, -OR¹⁰, SR¹⁰, -NR¹⁰R¹¹, -NC(O)R¹⁰-, optionally substituted C₁₋₆ alkyl, C₂₋₆ alkenyl or C₂₋₆ alkynyl, optionally substituted aryl or -C(O)R¹⁰, wherein the optional substituents in all cases are as defined above and, more preferably, are selected from hydroxyl, alkoxy, halogen or trialkylsilyl. More preferably, R² is hydrogen, halogen, cyano, methoxy, methylsulfanyl, methylamino, acetylamino or optionally substituted C₁₋₆ alkyl or C₂₋₆ alkynyl. Even more preferably, R² is hydrogen, chloro, bromo, cyano or methyl, 2- trimethylsilyl-ethynyl. Preferably, R² is hydrogen, halogen or C₁₋₄ alkyl. Most preferably, R² is hydrogen, chloro or methyl.

Preferably, X¹ is CH and R¹ and R² are hydrogen, halogen or C₁₋₄ alkyl. Preferably, X² is CH and R¹ and R² are hydrogen, halogen or C₁₋₄ alkyl. More preferably, X¹ and X² are CH and R¹ and R² are hydrogen, halogen or C₁₋₄ alkyl.

Preferably, R³ is hydrogen, hydroxyl, -C(O)R¹², -OR¹², -C(O)OR¹², -OC(O)R¹², -S(O)₂R¹², optionally substituted C₁₋₆ alkyl, C₂₋₆ alkenyl, C₃₋₆ allenyl, C₂₋₆ alkynyl or optionally substituted saturated cyclyl, wherein the optional substitutents in all cases are as defined above and, more preferably, are selected from cyano, halogen, hydroxyl, C₁₋₄ alkyl, C₂₋₄ alkenyl, alkoxy (optionally substituted by alkoxy or acyl), cyclyl, heterocyclyl, aryl, heteroaryl, -NH₂, trialkylsilyl, -C(O)R or -C(O)OR (wherein R is hydrogen, methyl or ethyl). Preferably, R³ is hydrogen, -C(O)R¹², -C(O)O R¹², -OC(O)R¹², -S(O)₂ R¹², optionally substituted C₁₋₆ alkyl, C₂₋₆ alkenyl, C₃₋₆ allenyl, C₂₋₆ alkynyl or optionally substituted saturated cyclyl. More preferably, R³ is hydrogen or optionally substituted C₁₋₆ alkyl, C₂₋₄ alkenyl, C₃₋₄ allenyl or C₂₋₄ alkynyl. Most preferably, R³ is hydrogen, cyanomethyl, aminoethyl, aminopropyl, prop-2-enyl, prop-2-ynyl, propa-1,2-dienyl, methoxymethyl, 2-fluoroethyl.

Preferably, X¹ is CH and R³ is hydrogen, -C(O)R¹², -C(O)OR¹², -OC(O)R¹², -S(O)₂ R¹², optionally substituted C₁₋₆ alkyl, C₂₋₆ alkenyl, C₃₋₆ allenyl, C₂₋₆ alkynyl or optionally substituted saturated cyclyl. Preferably, X² is CH and R³ is hydrogen, -C(O)R¹², -C(O)O R¹², -OC(O)R¹², -S(O)₂ R¹², optionally substituted C₁₋₆ alkyl, C₂₋₆ alkenyl, C₃₋₆ allenyl, C₂₋₆ alkynyl or optionally substituted saturated cyclyl. Preferably, R¹ and R² are hydrogen, halogen or C₁₋₄ alkyl and R³ is hydrogen, -C(O)R¹², -C(O)O R¹², -OC(O)R¹², -S(O)₂ R¹², optionally substituted C₁₋₆ alkyl, C₂₋₆ alkenyl, C₃₋₆ allenyl, C₂₋₆ alkynyl or optionally substituted saturated cyclyl. Preferably, X¹ and X² are CH and R³ is hydrogen, -C(O)R¹², -C(O)OR¹², -OC(O)R¹², -S(O)₂R¹², optionally substituted C₁₋₆ alkyl, C₂₋₆ alkenyl, C₃₋₆ allenyl, C₂₋₆ alkynyl or optionally substituted saturated cyclyl. More preferably, X¹ and X² are CH and R¹ and R² are hydrogen, halogen or C₁₋₄ alkyl and R³ is hydrogen, -C(O)R¹², -C(O)O R¹², -OC(O)R¹², -S(O)₂ R¹², optionally substituted C₁₋₆ alkyl, C₂₋₆ alkenyl, C₃₋₆ allenyl, C₂₋₆ alkynyl or optionally substituted saturated cyclyl.

Preferably, R⁴ is hydrogen, halogen, optionally substituted C₂₋₆ alkynyl or optionally substituted aryl or heteroaryl, wherein the optional substituents are as defined above and, more preferably, are selected from hydroxyl, halogen (in particular, fluoro or chloro), haloalkyl, acyl or C₁₋₄ alkyl (in particular, methyl). More preferably, R⁴ is optionally substituted phenyl or optionally substituted heteroaryl. Even more preferably, R⁴ is optionally substituted phenyl or thienyl. Even more preferably, R⁴ is phenyl, 3-methylphenyl, 3- trifluoromethylphenyl, 4-chlorophenyl, 2-fluorophenyl, 3-fluorophenyl, 4-fluorophenyl, 2,5- difluorophenyl, 3 -methyl-4- fluorophenyl or 5-chlorothien-2-yl. Most preferably, R⁴ is phenyl or 4-fluorophenyl.

Preferably, X¹ is CH and R⁴ is optionally substituted phenyl or thienyl. Preferably, X² is CH and R⁴ is optionally substituted phenyl or thienyl. Preferably, R¹ and R² are hydrogen, halogen or C₁₋₄ alkyl and R⁴ is optionally substituted phenyl or thienyl. Preferably, R³ is hydrogen, -C(O)R¹², -C(O)O R¹², -OC(O)R¹², -S(O)R¹², optionally substituted C₁₋₆ alkyl, C₂₋₆ alkenyl, C₃₋₆ allenyl, C₂₋₆ alkynyl or optionally substituted saturated cyclyl and R⁴ is optionally substituted phenyl or thienyl. Preferably, X¹ and X² are CH and R⁴ is optionally substituted phenyl or thienyl. More preferably, X¹ and X² are CH and R¹ and R² are hydrogen, halogen or C₁₋₄ alkyl and R⁴ is optionally substituted phenyl or thienyl. Preferably, X¹ and X² are CH and R¹ and R² are hydrogen, halogen or C₁₋₄ alkyl and R³ is hydrogen, -C(O)R¹², -C(O)O R¹², -OC(O)R¹², -S(O)R¹², optionally substituted C₁₋₆ alkyl, C₂₋₆ alkenyl, C₃₋₆ allenyl, C₂₋₆ alkynyl or optionally substituted saturated cyclyl and R⁴ is optionally substituted phenyl or thienyl.

Preferably R⁵ is hydrogen, halogen, optionally substituted C₁₋₆ alkyl, C₂₋₆ alkenyl or C₂₋₆ alkynyl, or forms an optionally substituted aryl, heteraryl, cyclyl or hetercycyl ring with R⁶, wherein the optional substituents in all cases are as defined above and, more preferably, are selected from halogen, cyano, hydroxyl, haloalkyl or C₁₋₄ alkyl. Preferably, the ring formed with R⁶ is a 5- or 6-membered heterocycle. More preferably, R⁵ is hydrogen or halogen. Most preferably, R⁵ is hydrogen.

Preferably R⁶ is hydrogen, halogen -C(O)OR¹⁸, -NR¹⁸R¹⁹, -N=CR²⁰ or forms an optionally substituted aryl, heteroaryl, cyclyl or heterocycyl ring with R⁵ as defined above. More preferably, R⁶ is hydrogen or -NR¹⁸R¹⁹. More preferably, R⁶ is -NHR¹⁹. More preferably, R⁶ is - NHC(O)R ²³. More preferably, R⁶ is hydrogen or -NR¹⁸R¹⁹.

Preferably, X¹ is CH and R⁶ is hydrogen or -NR¹⁸R¹⁹. Preferably, X² is CH and R⁶ is hydrogen or -NR¹⁸R¹⁹. Preferably, R¹ and R² are hydrogen, halogen or C₁₋₄ alkyl and R⁶ is hydrogen or - NR¹⁸R¹⁹. Preferably, R³ is hydrogen, -C(O)R¹², -C(O)O R¹², -OC(O)R¹², -S(O)R¹², optionally substituted C₁₋₆ alkyl, C₂₋₆ alkenyl, C₃₋₆ allenyl, C₂₋₆ alkynyl or optionally substituted saturated cyclyl and R⁶ is hydrogen or -NR¹⁸R¹⁹ Preferably, R⁴ is optionally substituted phenyl or thienyl and R⁶ is hydrogen or -NR¹⁸R¹⁹. Preferably, X¹ and X² are CH and R⁶ is hydrogen or -NR¹⁸R¹⁹. More preferably, X¹ and X² are CH and R¹ and R² are hydrogen, halogen or C₁₋₄ alkyl and R⁶ is hydrogen or -NR18R¹⁹. Preferably, X¹ and X² are CH and R¹ and R² are hydrogen, halogen or C ₁₋₄ alkyl and R³ is hydrogen, -C(O)R¹², -C(O)O R¹², -OC(O)R¹², -S(O)R¹², optionally substituted C₁₋₆ alkyl, C₂₋₆ alkenyl, C₃₋₆ allenyl, C₂₋₆ alkynyl or optionally substituted saturated cyclyl and R⁶ is hydrogen or -NR¹⁸R¹⁹. Preferably, X¹ and X² are CH and R¹ and R² are hydrogen, halogen or C₁₋₄ alkyl and R³ is hydrogen, -C(O)R¹², -C(O)O R¹², -OC(O)R¹², -S(O)R¹², optionally substituted C₁₋₆ alkyl, C₂₋₆ alkenyl, C₃₋₆ allenyl, C₂₋₆ alkynyl or optionally substituted saturated cyclyl and R⁴ is optionally substituted phenyl or thienyl and R⁶ is hydrogen or -NR¹⁸R¹⁹.

Preferably R⁷ is hydrogen, hydroxyl, cyano or optionally substituted C₁₋₆ alkyl, wherein the optional subsituents are as defined above and, more preferably, are selected from halogen, cyano, hydroxyl or haloalkyl. More preferably, R⁷ is hydrogen, hydroxyl or methyl. Most preferably, R⁷ is hydrogen.

Preferably, R¹⁰ R¹¹, R¹⁴, R¹⁵, R¹⁶ and R¹⁷ are, independently, hydrogen or optionally substituted C₁₋₆ alkyl, C₂₋₆ alkenyl or C₂₋₆ alkynyl, wherein the optional substituents are as defined above and, more preferably, are hydroxyl, halogen, cyano or alkoxy. More preferably R¹⁰, R¹¹, R¹⁴, R¹⁵, R¹⁶ and R¹⁷ are, independently, hydrogen or optionally substituted C₁₋₃ alkyl. Most preferably, R¹⁰ R¹¹, R¹⁴ R¹⁵, R¹⁶ and R¹⁷ are, independently, hydrogen, methyl or ethyl.

Preferably R¹² and R¹³ are, independently, optionally substituted C₁₋₆ alkyl, C₂₋₆ alkenyl or C₂₋₆ alkynyl or optionally substituted C₃₋₆ cyclyl, wherein the optional substituents in all cases are as defined above and, more preferably are hydroxyl, halogen, cyano, alkoxy, cyclyl (optionally substituted with hydroxyl or methyl), -C(O)OR, -OS(O)NRR' (wherein R and R' are, independently, hydrogen, alkyl, alkenyl or alkynyl). More preferably, R¹² and R¹³ are, independently, optionally substituted C₁₋₄ alkyl, C₂₋₄ alkenyl or C₂₋₄ alkynyl. Most preferably, R¹² and R¹³ are, independently, cyanomethyl, prop-2-enyl or prop-2-ynyl.

Preferably R¹⁸ is hydrogen, optionally substituted C₁₋₆ alkyl, C₂₋₆ alkenyl or C₂₋₆ alkynyl, - C(O)R²³, -C(O)OR²³, -S(O)₂R²³ or -C(O)NR²³R²⁴ or forms an optionally substituted heterocyclyl ring with R¹⁹, wherein the optional substituents in all cases are as defined above and, more preferably are selected from hydroxyl, cyano, halogen or alkoxy. More preferably, R¹⁸ is hydrogen, C₁₋₄ substituted alkyl, C₂₋₄ alkenyl, or C₂-₄ alkynyl. Preferably the C₁₋₄ alkyl group is ethyl or iso-propyl. Preferably, the C₂-₄ alkenyl group is propen-2-enyl. Preferably, the C₂₋₄ alkynyl group is prop-2ynyl or but-2-ynyl. Most preferably, R¹⁸ is hydrogen.

Preferably R¹⁹ is hydrogen, -C(S)R²³, -C(O)R²³, -C(O)OR²³, -S(O)₂R²³ or -C(O)NR²³R²⁴ optionally substituted C₁₋₆ alkyl, C₂₋₆ alkenyl or C₂₋₆ alkynyl, optionally substituted aryl, heteroaryl, cyclyl or heterocyclyl or forms an optionally substituted heterocyclyl ring with R¹⁸, wherein the optional substituents in all case are as defined above and, more preferably, are selected from hydroxyl, cyano, halogen, alkoxy, cyclyl or heterocyclyl. More preferably, R¹⁹ is hydrogen, -C(S)R²³, -C(O)R²³ or -C(O)OR²³ or optionally substituted C₁₋₄ alkyl. Preferably, the optionally substituted C₁₋₄ alkyl is iso-butyl. Most preferably, R¹⁹ is hydrogen, -C(O)R²³ or - C(O)OR²³_{.}

Preferably, R²⁰ is -NR²¹R²².

Preferably, R²¹ and R²² are, independently, hydrogen, optionally substituted C₁₋₄ alkyl or - C(O)OR²⁵, wherein the optional substituents are as defined above and, more preferably, are selected from hydroxyl, cyano, halogen, alkoxy, acyl, cyclyl or heterocyclyl. More preferably, R²¹ and R²² are, independently, hydrogen or optionally substituted C₁₋₄ alkyl. Most preferably, R²¹ and R²² are, independently, hydrogen, methyl or ethyl.

Preferably R²³ and R²⁴ are, independently, hydrogen, hydroxyl, optionally substituted C₁₋₆ alkyl, C₂₋₆ alkenyl or C₂₋₆ alkynyl or optionally substituted cyclyl or aryl, wherein the optional substituents in all cases are as defined above and, more preferably, are hydroxyl, halogen, cyano, C₁₋₄ alkyl, alkoxy, haloalkenyl, cyclyl or -C(O)OR (wherein R is cyclyl). Preferably, the aryl group is optionally substituted phenyl. More preferably, the aryl group is 3-halophenyl or 4-halophenyl. More preferably, R²³ and R²⁴ are, independently, hydrogen, optionally substituted C₁₋₆ alkyl or C₂₋₆ alkenyl or an optionally substituted saturated or mono-unsaturated cyclyl group. Even more preferably, R²³ and R²⁴ are, independently, optionally substituted C₁₋₆ alkyl or an optionally substituted C₃₋₆ saturated cyclyl group. Preferably the optionally substituted C₁₋₆ alkyl is methyl, ethyl or isopropyl. Preferably, the C₃₋₆ saturated cyclyl group is a cyclopropyl or cyclobutyl group, which may be substituted with one or more substituents being selected from cyano, halogen (preferably fluoro), C₁₋₄ alkyl (preferably methyl) or haloalkenyl.

Preferably, R²⁵ is C₁₋₄ alkyl. More preferably, R²⁵ is methyl, ethyl, propyl or 2- dimethylethyl.

In a particularly preferred embodiment, when R³ is hydrogen, R⁶ is other than hydrogen. More preferably, R³ is hydrogen and R⁶ is -NR¹⁸R¹⁹. More preferably, R³ is hydrogen and R⁶ is - NHR¹⁹. More preferably, R³ is hydrogen and R⁶ is -NHC(O)R²³.

In an alternative preferred embodiment, R⁶ is hydrogen and R³ is other than hydrogen. More preferably, R⁶ is hydrogen and R³ is optionally substituted C₁₋₆ alkyl, C₂₋₄ alkenyl, C₃₋₄ allenyl or C₂₋₄ alkynyl. Most preferably, R⁶ is hydrogen and R³ is cyanomethyl, aminoethyl, aminopropyl, prop-2-enyl, prop-2-ynyl, propa-1,2-dienyl, methoxymethyl, 2-fluoroethyl.

In a particular embodiment, compounds of formula (Ia) 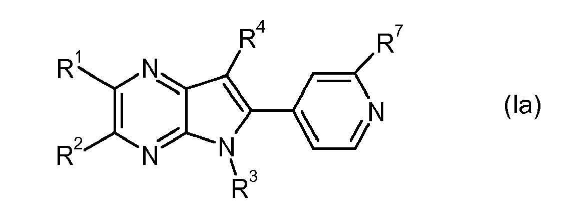 wherein R¹, R², R³, R⁴ and R⁷ are as defined above for preventing and/or controlling fungal infection in plants and/or plant propagation material and for reducing mycotoxin contamination in plants or plant material are described.
R¹ and R⁷ are preferably hydrogen, halogen or C₁₋₄ alkyl;

Preferably, R² is hydrogen, halogen, cyano, optionally substituted C₁₋₆ alkyl (in particular, optionally substituted C₁₋₄ alkyl and, most particularly, optionally substituted methyl or ethyl, wherein the optional substitutent is as defined above and more preferably is hydroxyl, e.g. 1-hydroxylethyl or -C(O)R¹⁰ and R¹⁰ is hydrogen or C₁₋₄ alkyl;
- R³: is preferably hydrogen, cyano, optionally substituted C₁₋₆ alkyl, C₂₋₆ alkenyl, C₃₋₆ allenyl or C₂₋₆ alkynyl, -NR¹²R¹³ or -C(O)R¹², wherein:
(a) the optional substituents on the alkyl, alkenyl and alkynyl groups are as defined above and, more preferably, are independently selected from halo, cyano, hydroxyl, alkoxy (optionally substituted by alkoxy or acyl), C₁₋₄ alkyl, C₂₋₄, alkenyl, cyclyl, heterocyclyl, heteroaryl, -C(O)R, -C(O)OR and -SR, wherein R is hydrogen, C₁₋₄ alkyl, C₂₋₄ alkenyl or C₂₋₄ alkynyl, and
(b) R¹² is optionally substituted alkyl, alkenyl, alkynyl or cyclyl, the optional substituents being as defined above and, more preferably, halo, cyano, hydroxyl, alkoxy, cyclyl, heterocyclyl, -C(O)R, -C(O)OR or -OS(O)NRR', wherein R and R' are, independently hydrogen or alkyl,
and
- R⁴: is optionally substituted aryl (in particular, phenyl or naphthyl) or heteroaryl (in particular, thienyl), the optional substituents being as defined above and, more preferably halogen or C₁₋₄ alkyl.

More preferably, R¹ is hydrogen, halogen or optionally substituted C₁₋₄ alkyl, R¹⁰ is methyl or ethyl; R² and R⁷ are, independently, hydrogen, methyl, ethyl or chloro; R³ is hydrogen or optionally substituted C₁₋₄ alkyl, C₂₋₄ alkenyl or C₂₋₄ alkynyl; and R⁴ is phenyl, which is optionally substituted by at least one substituent selected from halogen and C₁₋₄ alkyl (in particular, methyl).

Even more preferably, R¹, R² and R⁷ are each hydrogen; R³ is hydrogen, cyanomethyl, prop-2-enyl or prop-2-ynyl; and R⁴ is phenyl, 2- fluorophenyl, 3-fluorophenyl, 4-fluorophenyl, 4-chlorophenyl, 3-methylphenyl or 3-methyl- 4-fluorophenyl and most preferably is 4-fluorophenyl.
with the proviso that the compounds from the group consisting of
7-(4-fluorophenyl)-5-methyl-6-(4-pyridinyl)-5H-pyrrolo[2,3-b]pyrazine, 7-(3-chlorophenyl)-6-(2-chloro-4-pyridinyl)- 5H-pyrrolo[2,3-b]pyrazine, 7-(4-fluorophenyl)-3-methoxy-6-(4-pyridinyl)- 5H-pyrrolo[2,3-b]pyrazine, 7-(4-fluorophenyl)-N,N-dimethyl-6-(4-pyridinyl)- 5H-pyrrolo[2,3-b]pyrazin-3-amine, 5-ethyl-7-(4-fluorophenyl)-6-(4-pyridinyl)- 5H-pyrrolo[2,3-b]pyrazine, 7-(4-fluorophenyl)-N-methyl-6-(4-pyridinyl)- 5H-pyrrolo[2,3-b]pyrazine-5-propanamine, 3-chloro-6-(2-chloro-4-pyridinyl)-7-(4-fluorophenyl)- 5H-pyrrolo[2,3-b]pyrazine, 7-(4-fluorophenyl)-3-(methylthio)-6-(4-pyridinyl)- 5H-pyrrolo[2,3-b]pyrazine, 7-(4-fluorophenyl)-5-[3-(4-nitro-1H-imidazol-l-yl)propyl]-6-(4-pyridinyl)- 5H-pyrrolo[2,3-b]pyrazine, 7-(4-fluorophenyl)-5-[3-(2-nitro-1H-imidazol-1-yl)propyl]-6-(4-pyridinyl)- 5H-pyrrolo[2,3-b]pyrazine, 7-(4-fluorophenyl)-5-[3-(4-methyl-1-piperazinyl)propyl]-6-(4-pyridinyl)- 5H-pyrrolo[2,3-b]pyrazine, 7-(4-fluorophenyl)-5-[3-(4-morpholinyl)propyl]-6-(4-pyridinyl)- 5H-pyrrolo[2,3-b]pyrazine, N-[7-(4-fluorophenyl)-6-(4-pyridinyl)-5H-pyrrolo[2,3-b]pyrazin-3-yl]-acetamide, 6-(4-pyridinyl)- 5H-pyrrolo[2,3-b]pyrazine, 6-(2-bromo-4-pyridinyl)-7-(4-fluorophenyl)-5H-pyrrolo[2,3-b]pyrazine, 7-(4-fluorophenyl)-5-[3-(4-methyl-1H-imidazol-1-yl)propyl]-6-(4-pyridinyl)- 5H-pyrrolo[2,3-b]pyrazine, 5-(3-chloropropyl)-7-(4-fluorophenyl)-6-(4-pyridinyl)-5H-pyrrolo[2,3-b]pyrazine, 7-(4-fluorophenyl)-6-(4-pyridinyl)-5H-pyrrolo[2,3-b]pyrazine, 6-(2-chloro-4-pyridinyl)-7-(4-fluorophenyl)-5H-pyrrolo[2,3-b]pyrazine, 7-(4-fluorophenyl)-5-[3-(1H-imidazol-1-yl)propyl]-6-(4-pyridinyl)-5H-pyrrolo[2,3-b]pyrazine, 7-(4-fluorophenyl)-6-(4-pyridinyl)-5H-pyrrolo[2,3-b]pyrazine-5-butanenitrile are excluded.

In a particular embodiment, compounds of formula (Ib) : 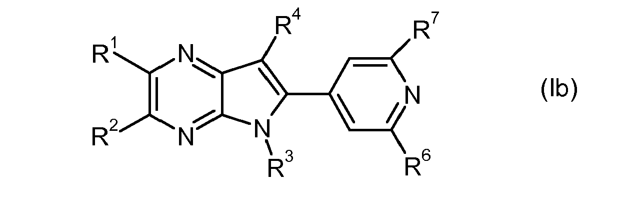 wherein R¹, R², R³, R^{4,} R⁶ and R⁷ are as defined above
for preventing and/or controlling fungal infection in plants and/or plant propagation material and for reducing mycotoxin contamination in plants or plant material are described
Preferably R¹ is hydrogen, halogen or C₁₋₄ alkyl;
- R²: is preferably hydrogen, halogen, methoxy or C₁₋₄ alkyl;
- R³: is preferably hydrogen, optionally substituted C₁₋₆ alkyl, C₂₋₆ alkenyl or C₂₋₆ alkynyl, - C(O)R¹² and R¹² is optionally substituted C₁₋₄ alkyl or cyclyl, the optional substituents in all cases being as defined above and, more preferably, halogen, cyano, hydroxyl, alkoxy, cyclyl, heterocyclyl, -NH₂, trialkylsilyl or C(O)OR, wherein R is hydrogen, C₁₋₄ alkyl, C₂₋₄ alkenyl or C₂₋₄ alkynyl;
- R⁴: is preferably optionally substituted aryl or heteroaryl, the optional substituents being as defined above and, more preferably halogen or C₁₋₄ alkyl;
- R⁶: is preferably halogen or -NR¹⁸R¹⁹ and
(i) R¹⁸ is hydrogen, -C(O)R²³, -C(O)OR²³ or optionally substituted C₁₋₄ alkyl, C₂₋₄ alkenyl or C₂₋₄ alkynyl and R²³ is optionally substituted C₁₋₄ alkyl and
(ii) R¹⁹ is hydrogen, optionally substituted C₁₋₄ alkyl, C₂₋₄ alkenyl or C₂₋₄ alkynyl, - C(S)R²³ -C(O)R²³ or -C(O)OR²³ and R²³ is hydrogen, optionally substituted C₁₋₄ alkyl, C₂₋₄ alkenyl, C₂₋₄ alkynyl or C₃₋₆ cyclyl;
- R⁷: is preferably hydrogen, halogen or C₁₋₄ alkyl.

More preferably, R¹ is hydrogen, chloro or C₁₋₄ alkyl; R² is hydrogen ,chloro, methoxy or methyl; R³ is hydrogen, optionally substituted C₁₋₄ alkyl, C₂₋₄ alkenyl or C₂₋₄ alkynyl; R⁴ is phenyl or thienyl, which are optionally substituted by at least one substituent selected from halogen and C₁₋₄ alkyl; R⁶ is halogen or -NR¹⁸R¹⁹ and R¹⁸ is hydrogen, prop-2- enyl or prop-2-ynyl and R¹⁹ is -C(O)R²³ and R²³ is hydrogen, methyl, ethyl, iso-propyl, 1- methylethyl, 1 - methylpropyl, 2-dimethylethyl, propyl, 1-methylethenyl, 2-methylprop-l- enyl, but-3-enyl, cyclopropyl, 1-methylcyclopropyl, 1-fluorocyclopropyl or cyclobutyl; R⁷ is hydrogen, chloro, fluoro or methyl.

Even more preferably, R¹, R² and R⁷ are hydrogen; R³ is hydrogen, cyanomethyl, prop-2-enyl or prop-2-ynyl; R⁴ is 2-fluorophenyl, 3-fluorophenyl, 4-fluorophenyl, 4-chlorophenyl, 3-methylphenyl or 3-methyl-4-fluorophenyl and most preferably is 4-fluorophenyl; R⁶ is -NR¹⁸R¹⁹ and R¹⁸ is hydrogen and R¹⁹ is -C(O)R²³ and R²³ is methyl, ethyl, iso-propyl, cyclopropyl, cyclobutyl or 1-methylcyclopropyl
with the proviso that the compounds from the group consisting of
N-[4-[7-(4-fluorophenyl)-4H-pyrrolo[2,3-b]pyrazin-6-yl]-2-pyridinyl]-glycine, Nl-[4-[7-(4-fluorophenyl)-5H-pyrrolo[2,3-b]pyrazin-6-yl]-2-pyridinyl]-1,2-ethanediamine, 4-[7-(4-fluorophenyl)-5H-pyrrolo[2,3-b]pyrazin-6-yl]-N-methyl-2-pyridinamine, 7-(4-fluorophenyl)-5-methyl-6-(4-pyridinyl)-5H-pyrrolo[2,3-b]pyrazine, 7-(3-chlorophenyl)-6-(2-chloro-4-pyridinyl)-5H-pyrrolo[2,3-b]pyrazine, N-[4-[7-(4-fluorophenyl)-5H-pyrrolo[2,3-b]pyrazin-6-yl]-2-pyridinyl]-1-piperidineacetamide, 7-(4-fluorophenyl)-3-methoxy-6-(4-pyridinyl)- 5H-pyrrolo[2,3-b]pyrazine, 7-(4-fluorophenyl)-N,N-dimethyl-6-(4-pyridinyl)- 5H-pyrrolo[2,3-b]pyrazin-3-amine, 2-[[4-[7-(4-fluorophenyl)-3-(methylamino)-5H-pyrrolo[2,3-b]pyrazin-6-yl]-2-pyridinyl]amino]-ethanol, N-[4-[7-(4-fluorophenyl)-5H-pyrrolo[2,3-b]pyrazin-6-yl]-2-pyridinyl]-4-methyl-1-piperazineacetamide, N-[4-[7-(4-fluorophenyl)-5H-pyrrolo[2,3-b]pyrazin-6-yl]-2-pyridinyl]- 1H-imidazole-1-acetamide, 7-(4-fluorophenyl)-N-methyl-6-[2-(methylamino)-4-pyridinyl]- 5H-pyrrolo[2,3-b]pyrazin-3-amine, 4-[7-(3-chlorophenyl)-5H-pyrrolo[2,3-b]pyrazin-6-yl]-N-[2-(methylthio)ethyl]- 2-pyridinamine, 5-ethyl-7-(4-fluorophenyl)-6-(4-pyridinyl)- 5H-pyrrolo[2,3-b]pyrazine, Nl-[4-[7-(4-fluorophenyl)-5H-pyrrolo[2,3-b]pyrazin-6-yl]-2-pyridinyl]- 1,3-propanediamine, 7-(4-fluorophenyl)-N-(3-methoxypropyl)-6-[2-[(3-methoxypropyl)amino]-4-pyridinyl]- 5H-pyrrolo[2,3-b]pyrazin-3-amine, 7-(4-fluorophenyl)-N-methyl-6-(4-pyridinyl)- 5H-pyrrolo[2,3-b]pyrazine-5-propanamine, N-[4-[3-(acetylamino)-7-(4-fluorophenyl)-5H-pyrrolo[2,3-b]pyrazin-6-yl]-2-pyridinyl]-acetamide, 3-chloro-6-(2-chloro-4-pyridinyl)-7-(4-fluorophenyl)- 5H-pyrrolo[2,3-b]pyrazine, 2-[[4-[7-(4-fluorophenyl)-5H-pyrrolo[2,3-b]pyrazin-6-yl]-2-pyridinyl]amino]-ethanol, N-[4-[3-[(3-aminopropyl)amino]-7-(4-fluorophenyl)-4H-pyrrolo[2,3-b]pyrazin-6-yl]-2-pyridinyl]- 1,3-propanediamine, 7-(4-fluorophenyl)-N-(2-methoxyethyl)-6-[2-[(2-methoxyethyl)amino]-4-pyridinyl]- 5H-pyrrolo[2,3-b]pyrazin-3-amine, 7-(4-fluorophenyl)-3-(methylthio)-6-(4-pyridinyl)- 5H-pyrrolo[2,3-b]pyrazine, 4-[[7-(4-fluorophenyl)-6-[2-[(4-hydroxybutyl)amino]-4-pyridinyl]-4H-pyrrolo [2,3-b]pyrazin-3-yl]amino]- 1-butanol, N-[4-[7-(4-fluorophenyl)-5H-pyrrolo[2,3-b]pyrazin-6-yl]-2-pyridinyl]-2-methoxy-acetamide, 7-(4-fluorophenyl)-5-[3-(4-nitro-1H-imidazol-1-yl)propyl]-6-(4-pyridinyl)- 5H-pyrrolo[2,3-b]pyrazine, 2-[[7-(4-fluorophenyl)-6-[2-[(2-hydroxyethyl)amino]-4-pyridinyl]-4H-pyrrolo[2,3-b]pyrazin-3-yl]amino]- ethanol, 2-(dimethylamino)-N-[4-[7-(4-fluorophenyl)-5H-pyrrolo[2,3-b]pyrazin-6-yl]-2-pyridinyl]-acetamide, 3-[[7-(4-fluorophenyl)-6-[2-[[(2-hydroxyethyl)methyl]amino]-4-pyridinyl]-4H-pyrrolo[2,3-b]pyrazin-3-yl]amino]-1-propanol, 7-(4-fluorophenyl)-5-[3-(2-nitro-1H-imidazol-1-yl)propyl]-6-(4-pyridinyl)- 5H-pyrrolo[2,3-b]pyrazine, 3-(dimethylamino)-N-[4-[7-(4-fluorophenyl)-5H-pyrrolo[2,3-b]pyrazin-6-yl]-2-pyridinyl]-propanamide, 7-(4-fluorophenyl)-5-[3-(4-methyl-1-piperazinyl)propyl]-6-(4-pyridinyl)- 5H-pyrrolo[2,3-b]pyrazine, 7-(4-fluorophenyl)-5-[3-(4-morpholinyl)propyl]-6-(4-pyridinyl)- 5H-pyrrolo[2,3-b]pyrazine, N-[4-[7-(4-fluorophenyl)-5H-pyrrolo[2,3-b]pyrazin-6-yl]-2-pyridinyl]-2-(methylamino)-acetamide, N-[7-(4-fluorophenyl)-6-(4-pyridinyl)-5H-pyrrolo[2,3-b]pyrazin-3-yl]-acetamide, 6-(4-pyridinyl)- 5H-pyrrolo[2,3-b]pyrazine, 6-(2-bromo-4-pyridinyl)-7-(4-fluorophenyl)-5H-pyrrolo[2,3-b]pyrazine, 7-(4-fluorophenyl)-5-[3-(4-methyl-1H-imidazol-1-yl)propyl]-6-(4-pyridinyl)- 5H-pyrrolo[2,3-b]pyrazine, 5-(3-chloropropyl)-7-(4-fluorophenyl)-6-(4-pyridinyl)-5H-pyrrolo[2,3-b]pyrazine, 7-(4-fluorophenyl)-6-(4-pyridinyl)-5H-pyrrolo[2,3-b]pyrazine, N-[4-[7-(4-fluorophenyl)-5H-pyrrolo[2,3-b]pyrazin-6-yl]-2-pyridinyl]-acetamide, 4-[7-(4-fluorophenyl)-5H-pyrrolo[2,3-b]pyrazin-6-yl]-2-pyridinamine, 6-(2-chloro-4-pyridinyl)-7-(4-fluorophenyl)-5H-pyrrolo[2,3-b]pyrazine, 3-[[4-[7-(4-fluorophenyl)-5H-pyrrolo[2,3-b]pyrazin-6-yl]-2-pyridinyl] amino]-1-propanol, 7-(4-fluorophenyl)-5- [3-(1H-imidazol-1-yl)propyl]-6-(4-pyridinyl)-5H-pyrrolo[2,3-b]pyrazine, 7-(4-fluorophenyl)-6-(4-pyridinyl)-5H-pyrrolo[2,3-b]pyrazine-5-butanenitrile are excluded.

In a particular embodiment, compounds of formula (Ic) 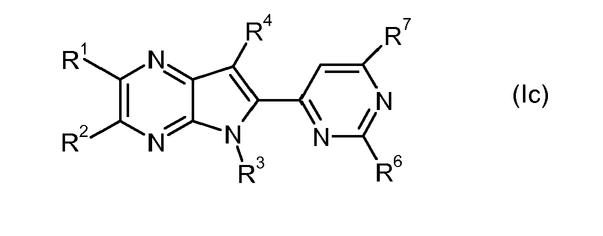 wherein R¹, R², R³, R⁴, R⁶ and R⁷ are as defined above for preventing and/or controlling fungal infection in plants and/or plant propagation material and for reducing mycotoxin contamination in plants or plant material are described

Preferably: R¹, R² and R⁷ are, independently, hydrogen, halogen or C₁₋₄ alkyl; R³ is hydrogen or optionally substituted C₁₋₆ alkyl, C₂₋₆ alkenyl or C₂₋₆ alkynyl, the optional substituents being as defined above and, more preferably, halogen or alkoxy; R⁴ is optionally substituted aryl, the optional subsituents being as defined above and, more preferably, halogen; and R⁶ is hydrogen, -SR¹⁸ or -NR¹⁸R¹⁹ wherein R¹⁸ is hydrogen or C₁₋₄ alkyl and R¹⁹ is optionally substituted alkyl, - C(S)R²³ or -C(O)R²³ and R²³ is hydrogen or C₁₋₄ alkyl.

More preferably R¹, R² and R⁷ are, independently, hydrogen, methyl, ethyl or chloro; R³ is hydrogen, haloalkyl, alkoxyalkyl, alkenyl or alkynyl; R⁴ is optionally substituted phenyl, the optional substituent being halogen; and R⁶ is hydrogen or -NR¹⁸R¹⁹ wherein R¹⁸ is hydrogen and R¹⁹ is 2-methoxy-1-methylethyl, -C(S)R²³ or -C(O)R²³ and R²³ is C₁₋₄ alkyl.

Even more preferably, R¹, R² and R⁷ are, independently, hydrogen; R³ is hydrogen, 2-fluoroethyl, methoxymethyl, prop-1,2-diene or prop-2-ynyl; R⁴ is fluorophenyl (in particular, 4-fluorophenyl); and R⁶ is -NR¹⁸R¹⁹ wherein R¹⁸ is hydrogen and R¹⁹ is - C(O)R²³ and R²³ methyl, ethyl, 1-methylethyl, 1-dimethylethyl or 3-methylpropyl.

The present invention provides a method for preventing and/or controlling fungal infection in plants and/or plant propagation material and a method for reducing of mycotoxin contamination in plants or plant material comprising applying to the plant or plant propagation material an effective amount of 4,7-diaza-indoles being compounds of formula (I): 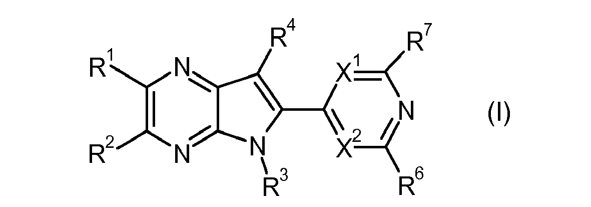 wherein:
- X¹: is N or CH;
- X₂: is N or CR⁵
- R¹ and R²: are, independently,:
(v) hydrogen, halogen, hydroxyl, cyano or nitro,
(vi) optionally substituted alkyl, alkenyl or alkynyl,
(vii) optionally substituted aryl, heteroaryl, cyclyl or heterocyclyl, or
(viii) C(O)R¹⁰, -C(O)NR¹⁰R¹¹, -C(S)NR¹⁰R¹¹, -C(NOR¹⁰)R¹¹, -C(O)OR¹⁰, -OR¹⁰, - SR¹⁰,-S(O)R¹⁰, -S(O)NR¹⁰R¹¹, -S(O)₂NR¹⁰R¹¹, -S(O)₂R¹⁰, -NR¹⁰R¹¹, -NC(O)R¹⁰, -P(O)(OR¹⁰)(OR¹¹) or-OP(O)(OR¹⁰)(OR¹¹);
- R³: is:
(i) hydrogen, hydroxyl, cyano or nitro,
(ii) optionally substituted alkyl, alkenyl, allenyl, alkynyl or haloalkyl, (iii) optionally substituted aryl, heteroaryl, cyclyl or heterocyclyl or heteroaralkyl or (iv) -C(O)R¹²_{,} -C(O)OR¹², -OR¹² -OC(O)R¹², -S(O)₂R¹² or -NR¹²R¹³;
- R⁴: is:
(v) hydrogen, halogen, hydroxyl, cyano or nitro,
(vi) (ii) optionally substituted alkyl, alkenyl, allenyl, alkynyl or haloalkyl,
(vii) (iii) optionally substituted aryl, heteroaryl, cyclyl or heterocyclyl or
(viii) -C(O)R¹⁴, -C(O)OR¹⁴, -C(NOR¹⁴)R¹⁵, -OR¹⁴, -SR¹⁴, -S(O)NR¹⁴R¹⁵, -S(O)₂R¹⁴, or -NR¹⁴R¹⁵;
- R⁵: is:
(iv) hydrogen, halogen, hydroxyl, cyano or nitro,
(v) optionally substituted alkyl, alkenyl or alkynyl,
(vi) -C(O)R¹⁶, -C(O)OR¹⁶, -OR¹⁶, -SR¹⁶, -S(O)R¹⁶, -S(O)NR¹⁶R¹⁷, -S(O)₂R¹⁶, or - NR¹⁶R¹⁷;
- R⁶: is hydrogen, halogen, cyano, -C(O)OR¹⁸, -SR¹⁸, -NR¹⁸R¹⁹, -C(O)NR¹⁹R¹⁹ or -N=CR²⁰,-C(=NR¹⁸)NR¹⁹R²⁰ or optionally substituted aryl, heteroaryl, cyclyl or heterocyclyl; ;
- R⁷: is hydrogen, halogen, hydroxyl, cyano, nitro or optionally substituted alkyl;
- R¹⁰, R¹¹, R¹⁴, R¹⁵ R¹⁶ and R¹⁷: , are, independently, hydrogen, halogen, hydroxyl, cyano, nitro, optionally substituted alkyl, alkoxy, alkenyl or alkynyl, or optionally substituted aryl, heteroaryl, cyclyl or heterocyclyl;
- R¹² and R¹³: are, independently, hydrogen, halogen, hydroxyl, cyano, nitro, -NR²¹ R²², optionally substituted alkyl, alkoxy, alkenyl or alkynyl, or optionally substituted aryl, heteroaryl, cyclyl or heterocyclyl;
- R¹⁸ and R¹⁹: are, independently,
(v) hydrogen,
(vi) optionally substituted alkyl, alkenyl or alkynyl,
(vii) optionally substituted aryl, heteroaryl, cyclyl or heterocyclyl, or
(viii) -C(S)R²³ -C(O)R²³ -SO₂R²³, -C(O)OR²³, -OR²³ or C(O)NR²3R²⁴;
- R²⁰: is hydroxyl, optionally substituted alkyl or alkoxy or -NR²¹R²², or -N=CR²¹R²²;
- R²¹ and R²²: are, independently, hydrogen, optionally substituted alkyl, alkenyl or alkynyl, optionally substituted cyclyl, heterocyclyl, aryl, or heteroaryl or aralkyl or -C(O)OR²⁵;
- R²³ and R²⁴: are, independently, hydrogen, hydroxyl, optionally substituted alkyl, alkenyl or alkynyl, or optionally substituted aryl, heteroaryl, cyclyl or heterocyclyl or aralkyl; and
- R²⁵: is optionally substituted alkyl, alkenyl or alkynyl;
and/or
independently, (i) R¹ and R² (ii) R³ and R⁵, (iii) R⁵ and R⁶, (iv) R⁵ and R¹⁸, (v) R⁵ and R¹⁹, (vi) R¹⁴ and R¹⁵ and (vii) R¹⁸ and R¹⁹ form an optionally substituted aryl, heteroaryl, cyclyl or heterocyclyl group containing from 5 to 18 ring atoms;
or a salt of N-oxide thereof.

The substituents R¹, R², R³ , R⁴, R⁵, R⁶, R⁷, R¹⁰, R¹¹, R¹², R¹³, R¹⁴, R¹⁵,R¹⁶,R¹⁷, R¹⁸, R¹⁹, R²⁰,R²¹, R²², R²³, R²⁴, and R²⁵ are as defined above also for the preferred embodiments.

A composition for preventing and/or controlling fungal infection in plants and/or plant propagation material and for reducing mycotoxin contamination in plants or plant material comprising the compounds according to formula (I) with the substituents as defined above are also described for the present invention.

A composition for preventing and/or controlling fungal infection in plants and/or plant propagation material comprising the compounds according to formula (I) with the substituents as defined above are also described for the present invention.

A composition for reducing mycotoxin contamination in plants or plant material comprising the compounds according to formula (I) with the substituents as defined above are also described for the present invention.

The present invention provides a method for preventing and/or controlling fungal infection in plants and/or plant propagation material and a method for the reduction of mycotoxin contamination in plants or plant material comprising applying to the plant or plant propagation material an effective amount of 4,7-diaza-indoles being compounds of formula (Ia), (Ib), (Ic), and (Id).

In a further aspect, the invention relates to a process for the preparation of a compound of formula (I) as claimed in claim 1.

Compounds of formula (I) can be prepared according to the following processes A to E:

### Process A

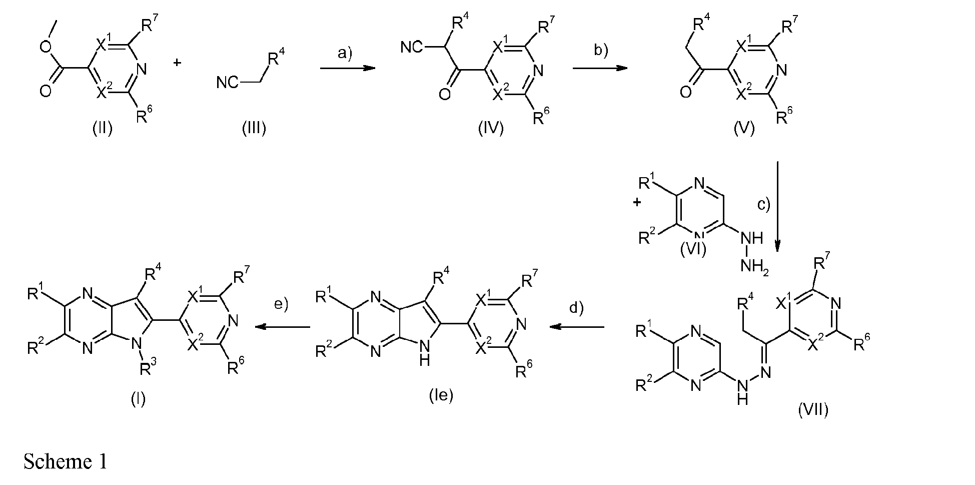

Compounds according to the invention can be prepared by methods known from the literature, as described for example in WO1999/20624 and in the literature cited therein.

The present invention provides a method for producing a compound according to formula (I), wherein as shown in scheme 1
in a first step (a) a compound of formula (II) 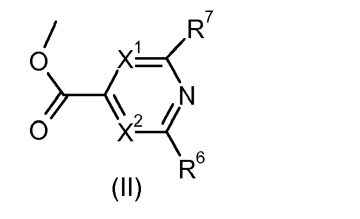 with R⁶ and R⁷ as defined above is reacted with a compound of formula (III) 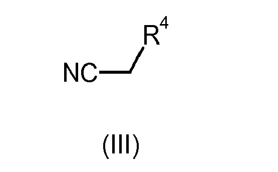 with R⁴ as defined above resulting in a compound of formula (IV) 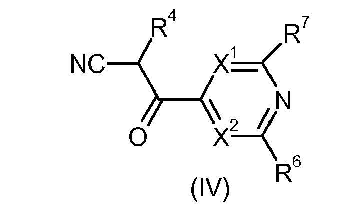 with R⁶, R⁷, and R⁴ as defined above,
the compound of formula (IV) is converted in a second step (b) to a ketone of formula (V) 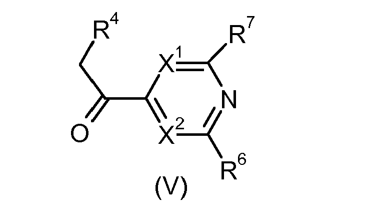 with R⁶, R⁷, and R⁴ as defined above,
the ketone of formula (V) is reacted in a third step (c) with a 2-hydrazinopyrazine of formula (VI) 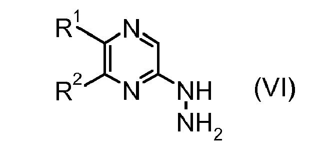 with R¹ and R² as defined above in the presence of a catalytic amount of an acid to a hydrazone of formula (VII) 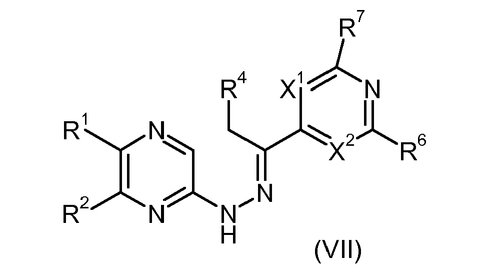 with R¹, R², R⁶, R⁷, and R⁴ as defined above,
the hydrazone of formula (VII) is converted in a fourth step (d) to a 5H-pyrrolo[2,3-b]pyrazine of formula (Ie) 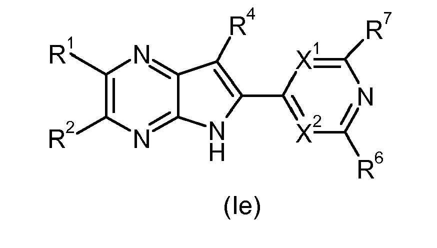 with R¹, R², R⁶, R⁷, and R⁴ as defined above,
by heating the hydrazone of formula (VII) in a high boiling solvent such as diethylene glycol to its boiling point,
in a fifth step the compound according to formula (Ie) is converted to a compound according to formula (I) in an aprotic solvent in the presence of a strong base.

### Step a)

Compounds of formula (IV) are prepared by reaction of an ester of formula (II) with an acetonitrile derivative of the formula (III) in the presence of a suitable base such as sodium ethoxide or potassium t-butoxide, each in its respective alcohol as solvent (see I. Lantos et al. J. Org. Chem. 53 (1988) 4223-4227).

In general, esters of formula (II) are commercially available or they can be prepared by methods well known in the art, for example starting from the free acids known in the literature under standard esterification reaction conditions.

Acetonitrile derivatives of the formula (III) such as 2-phenylacetonitrile, 4-fluorophenylacetonitrile, pyridylacetonitrile, and the like are commercially available or can be obtained by standard procedures..

### Step b)

Ketones of formula (V) are prepared by hydrolysis and decarboxylation of the cyano group of compounds of formula (IV) in a suitable aqueous acid such as hydrobromic acid. Alternatively, a compound of formula (V) can be prepared directly, by reacting the sodium salt of the corresponding free acid known in the literature with a Grignard reagent of formula R⁴CH₂MgX (where X is halogen).

### Step c)

The condensation of a ketone of formula (V) with a 2-hydrazinopyrazine of formula (VI) to the compound of the formula (VII) is described in J. Chem. Soc. Perkin I, 1361-63 (1976). Suitable solvents for the reaction are aromatic hydrocarbons such as toluene. The reaction can be carried out at temperatures between 50 and 200 °C. Suitable acids are such as p-toluenesulfonic acid.

The 2-hydrazinopyrazine of formula (VI) where R¹ and R² are both hydrogen is prepared by reacting 2-chloropyrazine with hydrazine under conditions well known in the art (see Eur. J. Med. Chem. 24(3), 249-57 (1989) and J. Heterocyclic Chem., 11, 697-701, (1974)). 2-Hydrazinopyrazines of formula (VI) with other substituents R¹ and R² can be prepared accordingly.

### Step d)

For other suitable cyclization reaction conditions see R. J. Sundberg, "Indoles" , Academic Press, San Diego, CA, 1996, p. 55.

### Step e)

A compound of formula (Ie) can be converted to a compound of formula (I) where R³ is not hydrogen by reacting it with an alkylating agent R³-Y where R3 is alkyl, alkenyl, alkynyl, heteroaralkyl, cyclyl, heterocyclyl or haloalkyl and where Y is a leaving group under alkylating conditions (such as halogen, mesylate, tosylate and the like) or an acylating or a sulfonylating agent R³-L where R³ is C(O)R¹², -C(O)OR¹² or -S(O)₂R¹² and where L is a leaving group under acylating reaction conditions such as halogen (preferably chlorine). The reaction is carried out in the presence of a strong base such as sodium hydride and in an aprotic organic solvent such as tetrahydrofuran, dimethylformamide, and the like.

### Process B

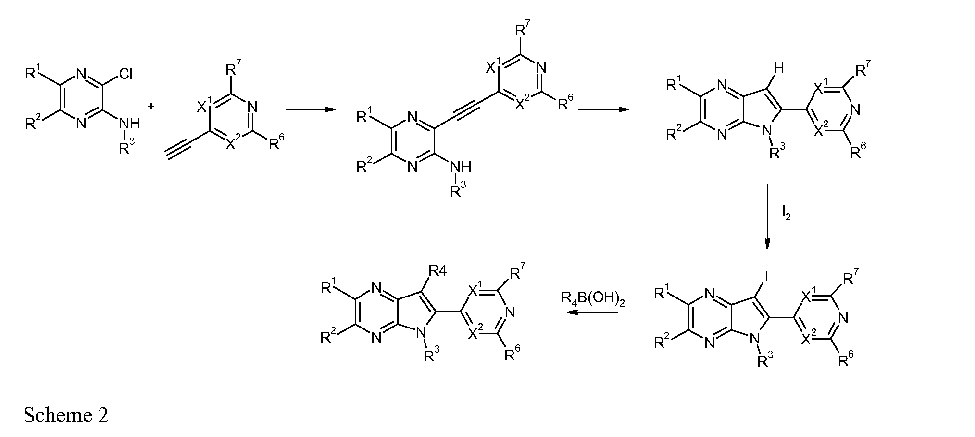

Compounds of of formula (I) can be synthesized also in analogy to other literature procedures (scheme 2): In a first step a *N-*alkylated o-chloroarylamine can be elaborated to *N-*alkyldiazaindoles via an one-pot process comprising a copper-free Sonogashira alkynylation and a base-mediated indolization reaction in analogy to M. McLaughlin, M. Palucki, I. W. Davies, Org. Lett., 2006, 8, 3307-3310 and M. C. de Mattos, S. Alatorre-Santamaria, V. Gotor-Fernández, V. Gotor, Synthesis, 2007, 2149-2152 and C. Harcken, Y. Ward, D. Thomson, D. Riether, Synlett 2005, 20, 3121-3125 and WO2007/017096. After that the compound can be iodinated followed by a Suzuki coupling reaction with a boronic acid in analogy to a procedure described in WO2008/022467.

### Process C

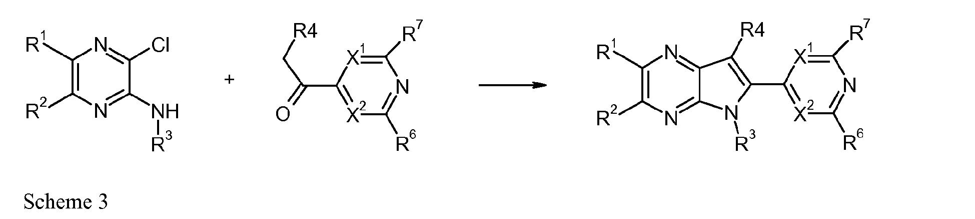

Another method for the synthesis of compounds of of formula (I) works by direct reaction of *N-*alkylated o-chloroarylamines with ketones (scheme 3) in analogy to a description in M. Nazare, C. Schneider, A. Lindenschmidt, D. W. Will, Angew. Chem. Int. Ed., 2004, 43, 4526-4528.

### Process D

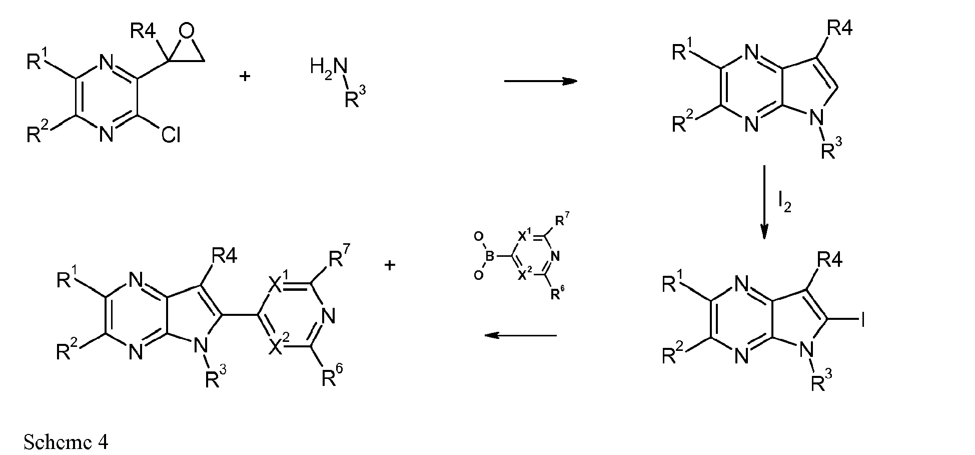

The synthesis of compounds of formula (I) is also possible starting from nicotinic acid derivatives which are first converted into epoxides followed by an epoxide-opening-cyclization-dehydration sequence (scheme 4) in analogy to H. Schirok, J. Org. Chem., 2006, 71, 5538-5545. The formed azaindole can be iodinated followed by a Suzuki coupling reaction with a boronic acid in analogy to a procedure described in WO2008/022467.

### Process E

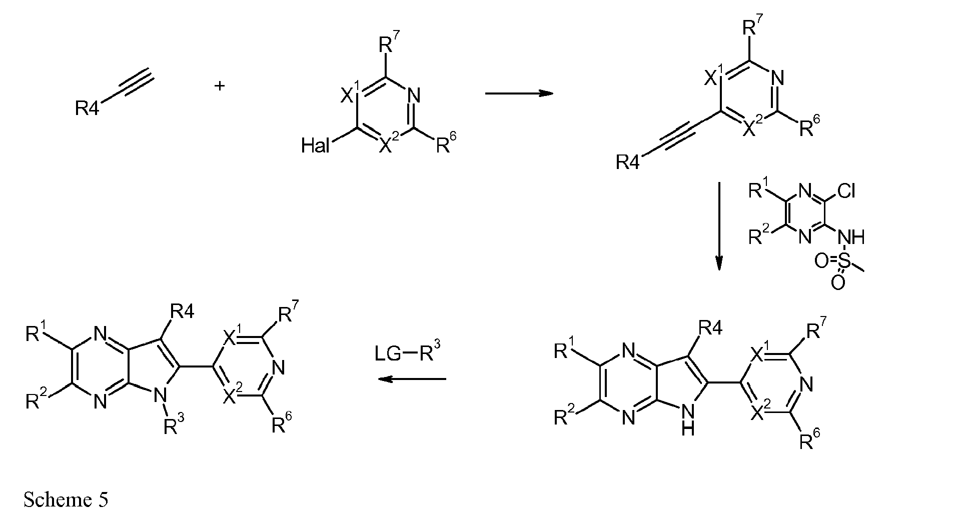

In a further alternative synthesis route to compounds of of formula (I) a diarylalkyne derivative reacts with a *N*-sulfonylated o-chloropyridazinylamine to form the diazaindol (scheme 5) as described in Tetrahedron Letters 2005, 46, 1845-1848. The alkylation of the formed azaindole and the Sonogashira coupling to the diarylalkyne can be performed by standard methods as described for example in WO1999/20624 or in Houben-Weyl, Methoden der Organischen Chemie [Methods in Organic Chemistry], Georg-Thieme-Verlag, Stuttgart.

The preparation is carried out under reaction conditions which are known and suitable for the abovementioned reactions. Other variants which are known per se, but not illustrated here in greater detail, may also be used.

If desired, the starting materials may also be formed in situ, in such a way that they are not isolated from the reaction mixture but immediately reacted further to give the compounds of the formula (I).

The starting materials of all processes are either commericially available or can be prepared by standard methods as described for example in Houben-Weyl, Methoden der Organischen Chemie [Methods in Organic Chemistry], Georg-Thieme-Verlag, Stuttgart.

Numerous fungi are serious pests of economically important agricultural crops. Further, crop contamination by fungal toxins is a major problem for agriculture throughout the world. Mycotoxins, such as aflatoxins, ochratoxins, patulin, fumonisins, zearalenones, and trichothecenes, are toxic fungal metabolites, often found in agricultural products that are characterized by their ability to cause health problems for humans and vertebrates. They are produced for example by different Fusarium and Aspergillus, Penicillium und Alternaria species.

Aflatoxins are toxins produced by Aspergillus species that grow on several crops, in particular on maize or corn before and after harvest of the crop as well as during storage. The biosynthesis of aflatoxins involves a complex polyketide pathway starting with acetate and malonate. One important intermediate is sterigmatocystin and O-methylsterigmatocystin which are direct precursors of aflatoxins. Important producers of aflatoxins are Aspergillus flavus, most strains of Aspergillus parasiticus, Aspergillus nomius, Aspergillus bombycis, Aspergillus pseudotamarii, Aspergillus ochraceoroseus, Aspergillus rambelli, Emericella astellata, Emericella venezuelensis, Bipolaris spp., Chaetomium spp., Farrowia spp., and Monocillium spp., in particular Aspergillus flavus and Aspergillus parasiticus (Plant Breeding (1999), 118, pp 1-16). There are also additional Aspergillus species known. The group of aflatoxins consists of more than 20 different toxins, in particular aflatoxin B1, B2, G1 and G2, cyclopiazonic acid (CPA).

Ochratoxins are mycotoxins produced by some *Aspergillus* species and *Penicilium* species, like *A. ochraceus, A. carbonarius* or *P. viridicatum,* Examples for Ochratoxins are ochratoxin A, B, and C. Ochratoxin A is the most prevalent and relevant fungal toxin of this group.

Fumonisins are toxins produced by Fusarium species that grow on several crops, mainly corn, before and after harvest of the crop as well as during storage. The diseases, Fusarium kernel, ear and stalk rot of corn, is caused by Fusarium verticillioides, F. subglutinans, F. *moniliforme,* and F. proliferatum. The main mycotoxins of these species are the fumonisins, of which more than ten chemical forms have been isolated. Examples for fumonisins are FB1, FB2 and FB3. In addition the above mentioned Fusarium species of corn can also produce the mycotoxins moniliformin and beauvericin. In particular Fusarium verticillioides is mentioned as an important pathogen of corn, this Fusarium species produces as the main mycotoxin fumonisins of the B-type.

Trichothecenes are those mycotoxins of primary concern which can be found in Fusarium diseases of small grain cereals like wheat, barley, rye, triticale, rice, sorghum and oat. They are sesquiterpene epoxide mycotoxins produced by species of Fusarium, Trichothecium, and Myrothecium and act as potent inhibitors of eukaryotic protein synthesis.

Some of these trichothecene producing Fusarium species also infect corn or maize.

Examples of trichothecene mycotoxins include T-2 toxin, HT-2 toxin, isotrichodermol, DAS, 3-deacetylcalonectrin, 3,15-dideacetylcalonectrin, scirpentriol, neosolaniol; 15-acetyldeoxynivalenol, 3-acetyldeoxynivalenol, nivalenol, 4-acetylnivalenol (fusarenone-X), 4,15-diacetylnivalenol, 4,7,15-acetylnivalenol, and deoxynivalenol (hereinafter "DON") and their various acetylated derivatives. The most common trichothecene in Fusarium head blight is DON produced for example by Fusarium graminearum and F. culmorum.

Another mycotoxin mainly produced by F. culmorum, F. graminearum and F. cerealis is zearalenone, a phenolic resorcyclic acid lactone that is primarily an estrogenic fungal metabolite.

Fusarium species that produce mycotoxins, such as fumonisins and trichothecenes, include F. acuminatum, F. crookwellense, F., verticillioides, F. culmorum, F. avenaceum, F. equiseti, F. moniliforme, F, graminearum (Gibberella zeae), F. lateritium, F. poae, F. sambucinum (G. pulicaris), F. proliferatum, F. subglutinans, F. sporotrichioides and other Fusarium species.

In contrast the species Microdochium nivale also a member of the so-called Fusarium complex is known to not produce any mycotoxins.

Both acute and chronic mycotoxicoses in farm animals and in humans have been associated with consumption of wheat, rye, barley, oats, rice and maize contaminated with Fusarium species that produce trichothecene mycotoxins. Experiments with chemically pure trichothecenes at low dosage levels have reproduced many of the features observed in moldy grain toxicoses in animals, including anemia and immunosuppression, haemorrage, emesis and feed refusal. Historical and epidemiological data from human populations indicate an association between certain disease epidemics and consumption of grain infected with Fusarium species that produce trichothecenes. In particular, outbreaks of a fatal disease known as alimentary toxic aleukia, which has occurred in Russia since the nineteenth century, have been associated with consumption of over-wintered grains contaminated with Fusarium species that produce the trichothecene T-2 toxin. In Japan, outbreaks of a similar disease called akakabi-byo or red mold disease have been associated with grain infected with Fusarium species that produce the trichothecene, DON. Trichothecenes were detected in the toxic grain samples responsible for recent human disease outbreaks in India and Japan. There exists, therefore, a need for agricultural methods for preventing, and crops having reduced levels of, mycotoxin contamination.

Further, mycotoxin-producing Fusarium species are destructive pathogens and attack a wide range of plant species. The acute phytotoxicity of mycotoxins and their occurrence in plant tissues also suggests that these mycotoxins play a role in the pathogenesis of Fusarium on plants. This implies that mycotoxins play a role in disease and, therefore, reducing their toxicity to the plant may also prevent or reduce disease in the plant. Further, reduction in disease levels may have the additional benefit of reducing mycotoxin contamination on the plant and particularly in grain where the plant is a cereal plant.

There is a need, therefore, to decrease the contamination by mycotoxins of plants and plant material before and/or after harvest and/or during storage.

It has also to be mentioned that breeding for fungal resistance in crops in contrast to insecticidal resistance is much more difficult. There have been several classical and transgenic breeding approaches, but obviously a high level of resistance is difficult to obtain.

As indicated above, it has now been found that the compounds of formula I are useful in reducing mycotoxin contamination when they are applied to a plant and/or any plant material and/or plant propagation material in an effective amount.

In a particular embodiment the fungi producing the mycotoxins are selected from the group of the following species: F. acuminatum, F. crookwellense, F., verticillioides, F. culmorum, F. avenaceum, F. equiseti, F. moniliforme, F. graminearum (Gibberella zeae), F. lateritium, F. poae, F. sambucinum (G. pulicaris), F. proliferatum, F. subglutinans and F. sporotrichioides, Aspergillus flavus, most strains of Aspergillus parasiticus and Aspergillus nomius, A. ochraceus, A. carbonarius or P. viridicatum.

In a very particular embodiment the fungi producing the mycotoxins are selected from the group of the following species: F., verticillioides, F. culmorum, F. moniliforme, F. graminearum (Gibberella zeae), F. proliferatum, Aspergillus flavus, most strains of Aspergillus parasiticus and Aspergillus nomius, A. ochraceus, A. carbonarius.

In a very particular embodiment the fungi producing the mycotoxins are selected from the group of the following species: F. verticillioides, F. proliferatum, F. graminearum (Gibberella zeae), Aspergillus flavus, and Aspergillus parasiticus.

In a very particular embodiment the fungi producing the mycotoxins are selected from the group of the following species: F. verticillioides, F. proliferatum, F. graminearum.

In a very particular embodiment the fungi producing the mycotoxins are selected from the group of the following species: Aspergillus flavus, and Aspergillus parasiticus.

In a particular embodiment the mycotoxins are selected from the following group: aflatoxins B1, B2, G1 and G2, ochratoxin A, B, C as well as T-2 toxin, HT-2 toxin, isotrichodermol, DAS, 3-deacetylcalonectrin, 3,15-dideacetylcalonectrin, scirpentriol, neosolaniol; zearalenone, 15-acetyldeoxynivalenol, nivalenol, 4-acetylnivalenol (fusarenone-X), 4,15-diacetylnivalenol, 4,7,15-acetylnivalenol, and deoxynivalenol (hereinafter "DON") and their various acetylated derivatives as well as fumonisins of the B-type as FB1, FB2, FB3.

In a very particular embodiment the mycotoxins are selected from the following group: aflatoxins B1, B2, G1 and G2, zearalenone, deoxynivalenol (hereinafter "DON") and their various acetylated derivatives as well as fumonisins of the B-type as FB1, FB2, FB3.

In a very particular embodiment the mycotoxins are selected from the following group: aflatoxins B1, B2, G1 and G2.

In a very particular embodiment the mycotoxins are selected from the following group: aflatoxins B1.

In a very particular embodiment the mycotoxins are selected from the following group: zearalenone, deoxynivalenol (hereinafter "DON") and their various acetylated derivatives.

In a very particular embodiment the mycotoxins are selected from the following group: fumonisins of the B-type as FB1, FB2, FB3.

In a particular embodiment of the invention plant or plant material before and/or after harvest and/or during storage has at least 10 % less mycotoxin, more preferable at least 20 % mycotoxin, more preferable at least 40 % mycotoxin, more preferable at least 50 % mycotoxin, more preferable at least 80 % mycotoxin contamination than plant or plant material before and/or after harvest and/or during storage which has not been treated.

In a particular embodiment of the invention plant or plant material before harvest has at least 10 % less mycotoxin, more preferable at least 20 % mycotoxin, more preferable at least 40 % mycotoxin, more preferable at least 50 % mycotoxin, more preferable at least 80 % mycotoxin contamination than plant or plant material before harvest which has not been treated.

In a particular embodiment of the invention plant or plant material after harvest has at least 10 % less mycotoxin, more preferable at least 20 % mycotoxin, more preferable at least 40 % mycotoxin, more preferable at least 50 % mycotoxin, more preferable at least 80 % mycotoxin contamination than plant or plant material after harvest which has not been treated.

In a particular embodiment of the invention plant or plant material during storage has at least 10 % less mycotoxin, more preferable at least 20 % mycotoxin, more preferable at least 40 % mycotoxin, more preferable at least 50 % mycotoxin, more preferable at least 80 % mycotoxin contamination than plant or plant during storage which has not been treated.

According to the invention all plants and plant material can be treated. By plants is meant all plants and plant populations such as desirable and undesirable wild plants, cultivars (including naturally occurring cultivars) and plant varieties (whether or not protectable by plant variety or plant breeder's rights). Cultivars and plant varieties can be plants obtained by conventional propagation and breeding methods which can be assisted or supplemented by one or more biotechnological methods such as by use of double haploids, protoplast fusion, random and directed mutagenesis, molecular or genetic markers or by bioengineering and genetic engineering methods including transgenic plants.

By plant material is meant all above ground and below ground parts and organs of plants such as shoot, leaf, flower, blossom and root, whereby for example leaves, needles, stems, branches, blossoms, fruiting bodies, fruits and seed as well as roots, corms and rhizomes are listed.

In a particular embodiment the plant material to be treated are leaves, shoots, flowers, grains, seeds.

In a particular embodiment the plant material to be treated are leaves, shoots, flowers, grains, seeds.

By 'plant propagation material' is meant generative and vegetative parts of a plant including seeds of all kinds (fruit, tubers, bulbs, grains etc), runners, pods, fruiting bodies, roots, rhizomes, cuttings, corms, cut shoots and the like.

Plant propagation material may also include plants and young plants which are to be transplanted after germination or after emergence from the soil.

Among the plants that can be protected by the method according to the invention, mention may be made of major field crops like corn, soybean, cotton, *Brassica* oilseeds such as *Brassica napus* (e.g. canola), *Brassica rapa, B. juncea* (e.g. mustard) and *Brassica carinata,* rice, wheat, sugarbeet, sugarcane, oats, rye, barley, millet, triticale, flax, vine and various fruits and vegetables of various botanical taxa such as *Rosaceae sp.* (for instance pip fruit such as apples and pears, but also stone fruit such as apricots, cherries, almonds and peaches, berry fruits such as strawberries), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (for instance banana trees and plantings), *Rubiaceae sp.* (for instance coffee), *Theaceae sp., Sterculiceae sp., Rutaceae sp.* (for instance lemons, oranges and grapefruit) *; Solanaceae sp.* (for instance tomatoes, potatoes, peppers, eggplant), *Liliaceae sp.,* Compositiae *sp.* (for instance lettuce, artichoke and chicory - including root chicory, endive or common chicory), *Umbelliferae sp.* (for instance carrot, parsley, celery and celeriac), *Cucurbitaceae sp.* (for instance cucumber - including pickling cucumber, squash, watermelon, gourds and melons), *Alliaceae* sp. (for instance onions and leek), Cruciferae *sp.* (for instance white cabbage, red cabbage, broccoli, cauliflower, brussel sprouts, pak choi, kohlrabi, radish, horseradish, cress, Chinese cabbage), Leguminosae *sp.* (for instance peanuts, peas and beans beans - such as climbing beans and broad beans), *Chenopodiaceae sp.* (for instance mangold, spinach beet, spinach, beetroots), *Malvaceae* (for instance okra), *Asparagaceae* (for instance asparagus); horticultural and forest crops; ornamental plants; as well as genetically modified homologues of these crops.

In a particular embodiment crops from the family of Poaceae which is comprised of wheat, oat, barley, rye, triticale, millet, corn, maize can be protected by the method of the invention.

The methods, compounds and compositions of the present invention are suitable for reducing mycotoxin contamination on a number of plants and their propagation material including, but not limited to the following target crops: vine, flaxcotton,cereals (wheat, barley, rye, oats, millet, triticale, maize (including field corn, pop corn and sweet corn), rice, sorghum and related crops); beet (sugar beet and fodder beet); sugar beet, sugar cane, leguminous plants (beans, lentils, peas, soybeans); oil plants (rape, mustard, sunflowers), *Brassica* oilseeds such as *Brassica napus* (e.g. canola), *Brassica rapa, B. juncea* (e.g. mustard) and *Brassica carinata;* cucumber plants (marrows, cucumbers, melons); fibre plants (cotton, flax, hemp, jute); vegetables (spinach, lettuce, asparagus, cabbages, carrots, eggplants, onions, pepper, tomatoes, potatoes, paprika, okra); plantation crops (bananas, fruit trees, rubber trees, tree nurseries), ornamentals (flowers, shrubs, broad-leaved trees and evergreens, such as conifers); as well as other plants such as vines, bushberries (such as blueberries), caneberries, cranberries, peppermint, rhubarb, spearmint, sugar cane and turf grasses including, but not limited to, cool-season turf grasses (for example, bluegrasses (Poa L.), such as Kentucky bluegrass (Poa pratensis L.), rough bluegrass (Poa trivialis L.), Canada bluegrass (Poa compressa L.) and annual bluegrass (Poa annua L.); bentgrasses (Agrostis L.), such as creeping bentgrass (Agrostis palustris Huds.), colonial bentgrass (Agrostis tenius Sibth.), velvet bentgrass (Agrostis canina L.) and redtop (Agrostis alba L.); fescues (Festuca L.), such as tall fescue (Festuca arundinacea Schreb.), meadow fescue (Festuca elatior L.) and fine fescues such as creeping red fescue (Festuca rubra L.), chewings fescue (Festuca rubra var. commutata Gaud.), sheep fescue (Festuca ovina L.) and hard fescue (Festuca longifolia); and ryegrasses (Lolium L.), such as perennial ryegrass (Lolium perenne L.) and annual (Italian) ryegrass (Lolium multiflorum Lam.)) and warm-season turf grasses (for example, Bermudagrasses (Cynodon L. C. Rich), including hybrid and common Bermudagrass; Zoysiagrasses (Zoysia Willd.), St. Augustinegrass (Stenotaphrum secundatum (Walt.) Kuntze); and centipedegrass (Eremochloa ophiuroides (Munro.) Hack.)); various fruits and vegetables of various botanical taxa such as *Rosaceae sp.* (for instance pip fruit such as apples and pears, but also stone fruit such as apricots, cherries, almonds and peaches, berry fruits such as strawberries), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (for instance banana trees and plantings), *Rubiaceae sp.* (for instance coffee), *Theaceae sp., Sterculiceae sp., Rutaceae sp.* (for instance lemons, oranges and grapefruit) *; Solanaceae sp.* (for instance tomatoes, potatoes, peppers, eggplant), *Liliaceae sp.,* Compositiae *sp.* (for instance lettuce, artichoke and chicory - including root chicory, endive or common chicory), *Umbelliferae sp.* (for instance carrot, parsley, celery and celeriac), *Cucurbitaceae sp.* (for instance cucumber - including pickling cucumber, squash, watermelon, gourds and melons), *Alliaceae* sp. (for instance onions and leek), Cruciferae *sp.* (for instance white cabbage, red cabbage, broccoli, cauliflower, brussel sprouts, pak choi, kohlrabi, radish, horseradish, cress, Chinese cabbage), Leguminosae *sp.* (for instance peanuts, peas and beans beans - such as climbing beans and broad beans), *Chenopodiaceae sp.* (for instance mangold, spinach beet, spinach, beetroots), *Malvaceae* (for instance okra), *Asparagaceae* (for instance asparagus); horticultural and forest crops; ornamental plants; as well as genetically modified homologues of these crops.

The method of treatment according to the invention can be used in the treatment of genetically modified organisms (GMOs), e.g. plants or seeds. Genetically modified plants (or transgenic plants) are plants in which a heterologous gene has been stably integrated into the genome. The expression "heterologous gene" essentially means a gene which is provided or assembled outside the plant and when introduced in the nuclear, chloroplastic or mitochondrial genome gives the transformed plant new or improved agronomic or other properties by expressing a protein or polypeptide of interest or by downregulating or silencing other gene(s) which are present in the plant (using for example, antisense technology, co suppression technology or RNA interference - RNAi - technology). A heterologous gene that is located in the genome is also called a transgene. A transgene that is defined by its particular location in the plant genome is called a transformation or transgenic event.

Depending on the plant species or plant cultivars, their location and growth conditions (soils, climate, vegetation period, diet), the treatment according to the invention may also result in superadditive ("synergistic") effects. Thus, for example, reduced application rates and/or a widening of the activity spectrum and/or an increase in the activity of the active compounds and compositions which can be used according to the invention, better plant growth, increased tolerance to high or low temperatures, increased tolerance to drought or to water or soil salt content, increased flowering performance, easier harvesting, accelerated maturation, higher harvest yields, bigger fruits, larger plant height, greener leaf color, earlier flowering, higher quality and/or a higher nutritional value of the harvested products, higher sugar concentration within the fruits, better storage stability and/or processability of the harvested products are possible, which exceed the effects which were actually to be expected.

At certain application rates, the active compound combinations according to the invention may also have a strengthening effect in plants. Accordingly, they are also suitable for mobilizing the defense system of the plant against attack by unwanted phytopathogenic fungi and/ or microorganisms and/or viruses. This may, if appropriate, be one of the reasons of the enhanced activity of the combinations according to the invention, for example against fungi. Plant-strengthening (resistance-inducing) substances are to be understood as meaning, in the present context, those substances or combinations of substances which are capable of stimulating the defense system of plants in such a way that, when subsequently inoculated with unwanted phytopathogenic fungi and/ or microorganisms and/or viruses, the treated plants display a substantial degree of resistance to these unwanted phytopathogenic fungi and/ or microorganisms and/or viruses. In the present case, unwanted phytopathogenic fungi and/ or microorganisms and/or viruses are to be understood as meaning phytopathogenic fungi, bacteria and viruses. Thus, the substances according to the invention can be employed for protecting plants against attack by the abovementioned pathogens within a certain period of time after the treatment. The period of time within which protection is effected generally extends from 1 to 10 days, preferably 1 to 7 days, after the treatment of the plants with the active compounds.

Plants and plant cultivars which are preferably to be treated according to the invention include all plants which have genetic material which impart particularly advantageous, useful traits to these plants (whether obtained by breeding and/or biotechnological means).

Plants and plant cultivars which are also preferably to be treated according to the invention are resistant against one or more biotic stresses, i.e. said plants show a better defense against animal and microbial pests, such as against nematodes, insects, mites, phytopathogenic fungi, bacteria, viruses and/or viroids.

Plants and plant cultivars which may also be treated according to the invention are those plants which are resistant to one or more abiotic stresses. Abiotic stress conditions may include, for example, drought, cold temperature exposure, heat exposure, osmotic stress, flooding, increased soil salinity, increased mineral exposure, ozon exposure, high light exposure, limited availability of nitrogen nutrients, limited availability of phosphorus nutrients, shade avoidance.

Plants and plant cultivars which may also be treated according to the invention, are those plants characterized by enhanced yield characteristics. Increased yield in said plants can be the result of, for example, improved plant physiology, growth and development, such as water use efficiency, water retention efficiency, improved nitrogen use, enhanced carbon assimilation, improved photosynthesis, increased germination efficiency and accelerated maturation. Yield can furthermore be affected by improved plant architecture (under stress and non-stress conditions), including but not limited to, early flowering, flowering control for hybrid seed production, seedling vigor, plant size, internode number and distance, root growth, seed size, fruit size, pod size, pod or ear number, seed number per pod or ear, seed mass, enhanced seed filling, reduced seed dispersal, reduced pod dehiscence and lodging resistance. Further yield traits include seed composition, such as carbohydrate content, protein content, oil content and composition, nutritional value, reduction in anti-nutritional compounds, improved processability and better storage stability.

Plants that may be treated according to the invention are hybrid plants that already express the characteristic of heterosis or hybrid vigor which results in generally higher yield, vigor, health and resistance towards biotic and abiotic stress factors. Such plants are typically made by crossing an inbred male-sterile parent line (the female parent) with another inbred male-fertile parent line (the male parent). Hybrid seed is typically harvested from the male sterile plants and sold to growers. Male sterile plants can sometimes (e.g. in corn) be produced by detasseling, i.e. the mechanical removal of the male reproductive organs (or males flowers) but, more typically, male sterility is the result of genetic determinants in the plant genome. In that case, and especially when seed is the desired product to be harvested from the hybrid plants it is typically useful to ensure that male fertility in the hybrid plants is fully restored. This can be accomplished by ensuring that the male parents have appropriate fertility restorer genes which are capable of restoring the male fertility in hybrid plants that contain the genetic determinants responsible for male-sterility. Genetic determinants for male sterility may be located in the cytoplasm. Examples of cytoplasmic male sterility (CMS) were for instance described in Brassica species. However, genetic determinants for male sterility can also be located in the nuclear genome. Male sterile plants can also be obtained by plant biotechnology methods such as genetic engineering. A particularly useful means of obtaining male-sterile plants is described in WO 1989/10396 in which, for example, a ribonuclease such as barnase is selectively expressed in the tapetum cells in the stamens. Fertility can then be restored by expression in the tapetum cells of a ribonuclease inhibitor such as barstar.

Plants or plant cultivars (obtained by plant biotechnology methods such as genetic engineering) which may be treated according to the invention are herbicide-tolerant plants, i.e. plants made tolerant to one or more given herbicides. Such plants can be obtained either by genetic transformation, or by selection of plants containing a mutation imparting such herbicide tolerance.

Herbicide-tolerant plants are for example glyphosate-tolerant plants, i.e. plants made tolerant to the herbicide glyphosate or salts thereof. Plants can be made tolerant to glyphosate through different means. For example, glyphosate-tolerant plants can be obtained by transforming the plant with a gene encoding the enzyme 5-enolpyruvylshikimate-3-phosphate synthase (EPSPS). Examples of such EPSPS genes are the AroA gene (mutant CT7) of the bacterium *Salmonella typhimurium,* the CP4 gene of the bacterium *Agrobacterium sp.,* the genes encoding a Petunia EPSPS, a Tomato EPSPS, or an Eleusine EPSPS (WO 2001/66704). It can also be a mutated EPSPS. Glyphosate-tolerant plants can also be obtained by expressing a gene that encodes a glyphosate oxido-reductase enzyme. Glyphosate-tolerant plants can also be obtained by expressing a gene that encodes a glyphosate acetyl transferase enzyme. Glyphosate-tolerant plants can also be obtained by selecting plants containing naturally-occurring mutations of the above-mentioned genes.

Other herbicide resistant plants are for example plants that are made tolerant to herbicides inhibiting the enzyme glutamine synthase, such as bialaphos, phosphinothricin or glufosinate. Such plants can be obtained by expressing an enzyme detoxifying the herbicide or a mutant glutamine synthase enzyme that is resistant to inhibition. One such efficient detoxifying enzyme is an enzyme encoding a phosphinothricin acetyltransferase (such as the bar or pat protein from Streptomyces species). Plants expressing an exogenous phosphinothricin acetyltransferase are described.

Further herbicide-tolerant plants are also plants that are made tolerant to the herbicides inhibiting the enzyme hydroxyphenylpyruvatedioxygenase (HPPD). Hydroxyphenylpyruvatedioxygenases are enzymes that catalyze the reaction in which para-hydroxyphenylpyruvate (HPP) is transformed into homogentisate. Plants tolerant to HPPD-inhibitors can be transformed with a gene encoding a naturally-occurring resistant HPPD enzyme, or a gene encoding a mutated HPPD enzyme. Tolerance to HPPD-inhibitors can also be obtained by transforming plants with genes encoding certain enzymes enabling the formation of homogentisate despite the inhibition of the native HPPD enzyme by the HPPD-inhibitor. Tolerance of plants to HPPD inhibitors can also be improved by transforming plants with a gene encoding an enzyme prephenate dehydrogenase in addition to a gene encoding an HPPD-tolerant enzyme.

Still further herbicide resistant plants are plants that are made tolerant to acetolactate synthase (ALS) inhibitors. Known ALS-inhibitors include, for example, sulfonylurea, imidazolinone, triazolopyrimidines, pyrimidinyloxy(thio)benzoates, and/or sulfonylaminocarbonyltriazolinone herbicides. Different mutations in the ALS enzyme (also known as acetohydroxyacid synthase, AHAS) are known to confer tolerance to different herbicides and groups of herbicides. The production of sulfonylurea-tolerant plants and imidazolinone-tolerant plants is described. Other imidazolinone-tolerant plants are also described. Further sulfonylurea- and imidazolinone-tolerant plants are also described.

Other plants tolerant to imidazolinone and/or sulfonylurea can be obtained by induced mutagenesis, selection in cell cultures in the presence of the herbicide or mutation breeding as described for soybeans, for rice, for sugar beet, for lettuce, or for sunflower.

Plants or plant cultivars (obtained by plant biotechnology methods such as genetic engineering) which may also be treated according to the invention are insect-resistant transgenic plants, i.e. plants made resistant to attack by certain target insects. Such plants can be obtained by genetic transformation, or by selection of plants containing a mutation imparting such insect resistance.

An "insect-resistant transgenic plant", as used herein, includes any plant containing at least one transgene comprising a coding sequence encoding:
1) an insecticidal crystal protein from *Bacillus thuringiensis* or an insecticidal portion thereof, such as the insecticidal crystal proteins listed at the *Bacillus thuringiensis* toxin nomenclature, online at: http://www.lifesci.sussex.ac.uk/Home/Neil_Crickmore/Bt/), or insecticidal portions thereof, e.g., proteins of the Cry protein classes CrylAb, CrylAc, Cry1F, Cry2Ab, Cry3Aa, or Cry3Bb or insecticidal portions thereof; or
2) a crystal protein from *Bacillus thuringiensis* or a portion thereof which is insecticidal in the presence of a second other crystal protein from *Bacillus thuringiensis* or a portion thereof, such as the binary toxin made up of the Cry34 and Cry35 crystal proteins; or
3) a hybrid insecticidal protein comprising parts of different insecticidal crystal proteins from *Bacillus thuringiensis,* such as a hybrid of the proteins of 1) above or a hybrid of the proteins of 2) above, e.g., the Cry1A.105 protein produced by corn event MON98034; or
4) a protein of any one of 1) to 3) above wherein some, particularly 1 to 10, amino acids have been replaced by another amino acid to obtain a higher insecticidal activity to a target insect species, and/or to expand the range of target insect species affected, and/or because of changes introduced into the encoding DNA during cloning or transformation, such as the Cry3Bb1 protein in corn events MON863 or MON88017, or the Cry3A protein in corn event MIR604;
5) an insecticidal secreted protein from *Bacillus thuringiensis* or *Bacillus cereus,* or an insecticidal portion thereof, such as the vegetative insecticidal (VIP) proteins listed at:
   http://www.lifesci.sussex.ac.uk/home/Neil Crickmore/Bt/vip.html,e.g., proteins from the VIP3Aa protein class; or
6) a secreted protein from *Bacillus thuringiensis* or *Bacillus cereus* which is insecticidal in the presence of a second secreted protein from *Bacillus thuringiensis* or *B. cereus,* such as the binary toxin made up of the VIP1A and VIP2A proteins; or
7) a hybrid insecticidal protein comprising parts from different secreted proteins from *Bacillus thuringiensis* or *Bacillus cereus,* such as a hybrid of the proteins in 1) above or a hybrid of the proteins in 2) above; or
8) a protein of any one of 1) to 3) above wherein some, particularly 1 to 10, amino acids have been replaced by another amino acid to obtain a higher insecticidal activity to a target insect species, and/or to expand the range of target insect species affected, and/or because of changes introduced into the encoding DNA during cloning or transformation (while still encoding an insecticidal protein), such as the VIP3Aa protein in cotton event COT102.

Of course, an insect-resistant transgenic plant, as used herein, also includes any plant comprising a combination of genes encoding the proteins of any one of the above classes 1 to 8. In one embodiment, an insect-resistant plant contains more than one transgene encoding a protein of any one of the above classes 1 to 8, to expand the range of target insect species affected when using different proteins directed at different target insect species, or to delay insect resistance development to the plants by using different proteins insecticidal to the same target insect species but having a different mode of action, such as binding to different receptor binding sites in the insect.

Plants or plant cultivars (obtained by plant biotechnology methods such as genetic engineering) which may also be treated according to the invention are tolerant to abiotic stresses. Such plants can be obtained by genetic transformation, or by selection of plants containing a mutation imparting such stress resistance. Particularly useful stress tolerance plants include:
a. plants which contain a transgene capable of reducing the expression and/or the activity of poly(ADP-ribose)polymerase (PARP) gene in the plant cells or plants.
b. plants which contain a stress tolerance enhancing transgene capable of reducing the expression and/or the activity of the PARG encoding genes of the plants or plants cells.
c. plants which contain a stress tolerance enhancing transgene coding for a plant-functional enzyme of the nicotinamide adenine dinucleotide salvage synthesis pathway including nicotinamidase, nicotinate phosphoribosyltransferase, nicotinic acid mononucleotide adenyl transferase, nicotinamide adenine dinucleotide synthetase or nicotine amide phosphoribosyltransferase.

Plants or plant cultivars (obtained by plant biotechnology methods such as genetic engineering) which may also be treated according to the invention show altered quantity, quality and/or storage-stability of the harvested product and/or altered properties of specific ingredients of the harvested product such as :
1) transgenic plants which synthesize a modified starch, which in its physical-chemical characteristics, in particular the amylose content or the amylose/amylopectin ratio, the degree of branching, the average chain length, the side chain distribution, the viscosity behaviour, the gelling strength, the starch grain size and/or the starch grain morphology, is changed in comparison with the synthesised starch in wild type plant cells or plants, so that this is better suited for special applications. Said transgenic plants synthesizing a modified starch are disclosed.
2) transgenic plants which synthesize non starch carbohydrate polymers or which synthesize non starch carbohydrate polymers with altered properties in comparison to wild type plants without genetic modification. Examples are plants producing polyfructose, especially of the inulin and levan-type, plants producing alpha 1,4 glucans, plants producing alpha-1,6 branched alpha-1,4-glucans, plants producing alternan,
3) transgenic plants which produce hyaluronan.

Plants or plant cultivars (that can be obtained by plant biotechnology methods such as genetic engineering) which may also be treated according to the invention are plants, such as cotton plants, with altered fiber characteristics. Such plants can be obtained by genetic transformation, or by selection of plants contain a mutation imparting such altered fiber characteristics and include:
a) Plants, such as cotton plants, containing an altered form of cellulose synthase genes,
b) Plants, such as cotton plants, containing an altered form of rsw2 or rsw3 homologous nucleic acids,
c) Plants, such as cotton plants, with increased expression of sucrose phosphate synthase,
d) Plants, such as cotton plants, with increased expression of sucrose synthase,
e) Plants, such as cotton plants, wherein the timing of the plasmodesmatal gating at the basis of the fiber cell is altered, e.g. through downregulation of fiberselective β 1,3-glucanase,
f) Plants, such as cotton plants, having fibers with altered reactivity, e.g. through the expression of N-acteylglucosaminetransferase gene including nodC and chitinsynthase genes.

Plants or plant cultivars (that can be obtained by plant biotechnology methods such as genetic engineering) which may also be treated according to the invention are plants, such as oilseed rape or related Brassica plants, with altered oil profile characteristics. Such plants can be obtained by genetic transformation or by selection of plants contain a mutation imparting such altered oil characteristics and include:
a) Plants, such as oilseed rape plants, producing oil having a high oleic acid content,
b) Plants such as oilseed rape plants, producing oil having a low linolenic acid content,
c) Plant such as oilseed rape plants, producing oil having a low level of saturated fatty acids.

Particularly useful transgenic plants which may be treated according to the invention are plants which comprise one or more genes which encode one or more toxins, such as the following which are sold under the trade names YIELD GARD® (for example maize, cotton, soya beans), KnockOut® (for example maize), BiteGard® (for example maize), Bt-Xtra® (for example maize), StarLink® (for example maize), Bollgard® (cotton), Nucotn® (cotton), Nucotn 33B®(cotton), NatureGard® (for example maize), Protecta® and NewLeaf® (potato). Examples of herbicide-tolerant plants which may be mentioned are maize varieties, cotton varieties and soya bean varieties which are sold under the trade names Roundup Ready@ (tolerance to glyphosate, for example maize, cotton, soya bean), Liberty Link® (tolerance to phosphinotricin, for example oilseed rape), IMI® (tolerance to imidazolinones) and STS® (tolerance to sulphonylureas, for example maize). Herbicide-resistant plants (plants bred in a conventional manner for herbicide tolerance) which may be mentioned include the varieties sold under the name Clearfield® (for example maize).

Particularly useful transgenic plants which may be treated according to the invention are plants containing transformation events, or combination of transformation events, that are listed for example in the databases from various national or regional regulatory agencies (see for example http://gmoinfo.jrc-it/gmp_browse.aspx and http://www.agbios.com/dbase.php).

When used in the methods of the invention, the compounds of formula I may be in unmodified form or, preferably, formulated together with carriers and adjuvants conventionally employed in the art of formulation.

The invention therefore also relates to a composition for the control of mycotoxin contamination comprising a compound of formula (I) as defined above and an agriculturally acceptable support, carrier or filler.

According to the invention, the term "support" denotes a natural or synthetic, organic or inorganic compound with which the active compound of formula (I) is combined or associated to make it easier to apply, notably to the parts of the plant. This support is thus generally inert and should be agriculturally acceptable. The support may be a solid or a liquid. Examples of suitable supports include clays, natural or synthetic silicates, silica, resins, waxes, solid fertilisers, water, alcohols, in particular butanol, organic solvents, mineral and plant oils and derivatives thereof. Mixtures of such supports may also be used.

The composition according to the invention may also comprise additional components. In particular, the composition may further comprise a surfactant. The surfactant can be an emulsifier, a dispersing agent or a wetting agent of ionic or non-ionic type or a mixture of such surfactants. Mention may be made, for example, of polyacrylic acid salts, lignosulphonic acid salts, phenolsulphonic or naphthalenesulphonic acid salts, polycondensates of ethylene oxide with fatty alcohols or with fatty acids or with fatty amines, substituted phenols (in particular alkylphenols or arylphenols), salts of sulphosuccinic acid esters, taurine derivatives (in particular alkyl taurates), phosphoric esters of polyoxyethylated alcohols or phenols, fatty acid esters of polyols, and derivatives of the present compounds containing sulphate, sulphonate and phosphate functions. The presence of at least one surfactant is generally essential when the active compound and / or the inert support are water-insoluble and when the vector agent for the application is water. Preferably, surfactant content may be comprised from 5% to 40% by weight of the composition.

Colouring agents such as inorganic pigments, for example iron oxide, titanium oxide, ferrocyanblue, and organic pigments such as alizarin, azo and metallophthalocyanine dyes, and trace elements such as iron, manganese, boron, copper, cobalt, molybdenum and zinc salts can be used.

Optionally, other additional components may also be included, e.g. protective colloids, adhesives, thickeners, thixotropic agents, penetration agents, stabilisers, sequestering agents. More generally, the active compounds can be combined with any solid or liquid additive, which complies with the usual formulation techniques.

In general, the composition according to the invention may contain from 0.05 to 99% by weight of active compounds, preferably from 10 to 70% by weight.

The combination or composition according to the invention can be used as such, in form of their formulations or as the use forms prepared therefrom, such as aerosol dispenser, capsule suspension, cold fogging concentrate, dustable powder, emulsifiable concentrate, emulsion oil in water, emulsion water in oil, encapsulated granule, fine granule, flowable concentrate for seed treatment, gas (under pressure), gas generating product, granule, hot fogging concentrate, macrogranule, microgranule, oil dispersible powder, oil miscible flowable concentrate, oil miscible liquid, paste, plant rodlet, powder for dry seed treatment, seed coated with a pesticide, soluble concentrate, soluble powder, solution for seed treatment, suspension concentrate (flowable concentrate), ultra low volume (ULV) liquid, ultra low volume (ULV) suspension, water dispersible granules or tablets, water dispersible powder for slurry treatment, water soluble granules or tablets, water soluble powder for seed treatment and wettable powder.

The treatment of plants and plant parts with the active compound combination according to the invention is carried out directly or by action on their environment, habitat or storage area by means of the normal treatment methods, for example by watering (drenching), drip irrigation, spraying, atomizing, broadcasting, dusting, foaming, spreading-on, and as a powder for dry seed treatment, a solution for seed treatment, a water-soluble powder for seed treatment, a water-soluble powder for slurry treatment, or by encrusting.

These compositions include not only compositions which are ready to be applied to the plant or seed to be treated by means of a suitable device, such as a spraying or dusting device, but also concentrated commercial compositions which must be diluted before application to the crop.

The active compounds within the composition according to the invention can be employed for reducing mycotoxin contamination in crop protection or in the protection of materials.

Within the composition according to the invention, bactericide compounds can be employed in crop protection for example for controlling Pseudomonadaceae, Rhizobiaceae, Enterobacteriaceae, Corynebacteriaceae and Streptomycetaceae.

The composition according to the invention can be used to curatively or preventively reduce the mycotoxin contamination of plants or crops. Thus, according to a further aspect of the invention, there is provided a method for curatively or preventively reduce the mycotoxin contamination of comprising the use of a composition comprising a compound according to formula (I) according to the invention by application to the seed, the plant or to the fruit of the plant or to the soil in which the plant is growing or in which it is desired to grow.

Suitably, the active ingredient may be applied to plant propagation material to be protected by impregnating the plant propagation material, in particular, seeds, either with a liquid formulation of the fungicide or coating it with a solid formulation. In special cases, other types of application are also possible, for example, the specific treatment of plant cuttings or twigs serving propagation.

The present invention will now be described by way of the following non-limiting examples. Those skilled in the art will promptly recognize appropriate variations from the procedures both as to reactants and as to reaction conditions and techniques

### EXAMPLES

Compound 1 - Preparation of 7-(4-fluorophenyl)-6-(pyridin-4-yl)-5H-Pyrrolo[2,3-b]pyrazine, following procedures described in scheme 1

Step 1: To a suspension of hydrazinopyrazine (400 mg, 3.57 mmol) in 50 ml Toluene was added 2(4-fluorphenyl)-1-(pyridin-4y1)ethanone (770 mg, 3.57 mmol) (J. Med Chem. 2003, 46, 4702) and p-toluenesulfonic acid (50 mg). The reaction mixture was refluxed with azeotropic removal of water. After 16 h, the reaction mixture was concentrated in vacuo to give the crude pyrazinylhydrazone (1.35 g) which was used in the next step without further purification.

Step 2: The crude pyrazinylhydrazone (1.35 g) obtained in step 2 was suspended in 12 ml diethylene glycol and the reaction mixture was heated at reflux. After 1.5 h the reaction mixture was cooled and poured into water. The product was extracted with t-butylmethylether and the organic layer was washed with brine, dried over MgSO₄ und concentrated in vacuo. The crude product was purified by chromatographie on silica gel (ethyl acetate/cyclohexane) to give 196 mg (13%) 7-(4-fluorophenyl)-6-(pyridin-4-yl)-5H-pyrrolo[2,3-b]pyrazine (logP (pH3.5): 1.33; 1H-NMR (400 MHz, DMSO-d): δ 8.61 (d, 2H), 8.46 (d, 1H), 8.35 (d, 1H), 7.51 (m, 4H), 7.24 (dd, 2H), 4.35 (s, 1H).

Compound 2: Preparation of 7-(4-fluorophenyl)-5-(prop-2-ylyl)-6-(pyridin-4-yl)-5H-pyrrolo[2,3-b]pyrazine following procedures described in scheme 1

Sodium hydride (15 mg, 0.37 mmole 60% in oil) is added to a solution of 120 mg (0.34 mmole) of 7-(4-fluorophenyl)-6-(pyridin-4-yl)-5H-pyrrolo[2,3-b]pyrazine (prepared as described in example 1) in 10 mL of dimethylformamide at room temperature. After 30 min. at room temperature, a solution of 56 mg (0.37 mmol) of propargylbromide in 0.5 mL of DMF is added to the reaction mixture. After 45 min. at room temperature, the reaction mixture is poured into ammonium chloride solution and extracted with ethyl acetate. The organic phase is washed with water and dried over magnesium sulfate. The solid residue after concentration is was purified by chromatographie on silica gel (ethyl acetate/cyclohexane) to give 72mg (64%) of 7-(4-fluorophenyl)-5-(prop-2-ylyl)-6-(pyridin-4-yl)-5H-pyrrolo[2,3-b]pyrazine. (logP (pH3.5): 2.22; ¹H-NMR (400 MHz, DMSO-d): δ = 8.69 (d, 2H), 8.52 (d, 1H), 8.37 (d, 1H), 7.47 (m, 4H), 7.05 (dd, 2H), 5.00 (d, 2H), 2.47 (t, 1H).

Although the invention has been described with reference to preferred embodiments and examples thereof, the scope of the present invention is not limited only to those described embodiments. As will be apparent to persons skilled in the art, modifications and adaptations to the above-described invention can be made without departing from the spirit and scope of the invention, which is defined and circumscribed by the appended claims. All publications cited herein are hereby incorporated by reference in their entirety for all purposes to the same extent as if each individual publication were specifically and individually indicated to be so incorporated by reference.

### Example A

### Alternaria test (tomato) / preventive

| | |
|---|---|
| Solvent: | 49 parts by weight ofN, N - Dimethylformamide |
| Emulsifier: | 1 part by weight of Alkylarylpolyglycolether |

To produce a suitable preparation of active compound, 1 part by weight of active compound is mixed with the stated amounts of solvent and emulsifier, and the concentrate is diluted with water to the desired concentration.

To test for preventive activity, young plants are sprayed with the preparation of active compound at the stated rate of application. One day after this treatment, the plants are inoculated with an aqueous spore suspension of ***Alternaria solani.*** The plants remain for one day in an incubation cabinet at approximately 22°C and a relative atmospheric humidity of 100%. Then the plants are placed in an incubation cabinet at approximately 20°C and a relative atmospheric humidity of 96%.

The test is evaluated 7 days after the inoculation. 0% means an efficacy which corresponds to that of the control while an efficacy of 100% means that no disease is observed.

In this test the compounds 1 and 2 according to the invention of the following structures showed efficacy of 70% or even higher at a concentration of 500ppm of active ingredient.

### Example B

### Pyrenophora test (barley) / preventive

| | |
|---|---|
| Solvent: | 49 parts by weight ofN, N - Dimethylformamide |
| Emulsifier: | 1 part by weight of Alkylarylpolyglycolether |

To produce a suitable preparation of active compound, 1 part by weight of active compound is mixed with the stated amounts of solvent and emulsifier, and the concentrate is diluted with water to the desired concentration.

To test for preventive activity, young plants are sprayed with the preparation of active compound at the stated rate of application. One day after this treatment, the plants are inoculated with an aqueous spore suspension of ***Pyrenophora teres**.* The plants remain for 48 hours in an incubation cabinet at 22°C and a relative atmospheric humidity of 100%. Then the plants are placed in a greenhouse at a temperature of approximately 20°C and a relative atmospheric humidity of approximately 80%.

The test is evaluated 7-9 days after the inoculation. 0% means an efficacy which corresponds to that of the control while an efficacy of 100% means that no disease is observed.

In this test the compounds 1 and 2according to the invention of the following structures showed efficacy of 70% or even higher at a concentration of 500ppm of active ingredient.

### Example C

### Venturia test (apples) / protective

| | |
|---|---|
| Solvent: | 24,5 parts by weight of acetone |
| | 24,5 parts by weight of dimethylacetamide |
| Emulsifier: | 1 part by weight of alkylaryl polyglycol ether |

To produce a suitable preparation of active compound, 1 part by weight of active compound is mixed with the stated amounts of solvent and emulsifier, and the concentrate is diluted with water to the desired concentration.

To test for protective activity, young plants are sprayed with the preparation of active compound at the stated rate of application. After the spray coating has dried on, the plants are inoculated with an aqueous conidia suspension of the causal agent of apple scab (Venturia inaequalis) and then remain for 1 day in an incubation cabinet at approximately 20°C and a relative atmospheric humidity of 100 %.

The plants are then placed in a greenhouse at approximately 21°C and a relative atmospheric humidity of approximately 90 %.

The test is evaluated 10 days after the inoculation. 0% means an efficacy which corresponds to that of the control, while an efficacy of 100% means that no disease is observed.

In this test the following Compound 2 according to the invention showed efficacy of 70% or even higher at a concentration of 100ppm of active ingredient.

### Example D

### Botrytis test (beans) / protective

| | |
|---|---|
| Solvent: | 24,5 parts by weight of acetone |
| | 24,5 parts by weight of dimethylacetamide |
| Emulsifier: | 1 part by weight of alkylaryl polyglycol ether |

To produce a suitable preparation of active compound, 1 part by weight of active compound is mixed with the stated amounts of solvent and emulsifier, and the concentrate is diluted with water to the desired concentration.

To test for protective activity, young plants are sprayed with the preparation of active compound. After the spray coating has dried on, 2 small pieces of agar covered with growth of Botrytis cinerea are placed on each leaf. The inoculated plants are placed in a darkened chamber at 20°C and a relative atmospheric humidity of 100%.

2 days after the inoculation, the size of the lesions on the leaves is evaluated. 0% means an efficacy which corresponds to that of the control, while an efficacy of 100% means that no disease is observed.

In this test the following Compound 2 according to the invention showed efficacy of 70% or even higher at a concentration of 250ppm of active ingredient.

### Example E

### Fusarium graminearum-test (barley) / preventive

| | |
|---|---|
| Solvent: | 49 parts by weight of n,n-dimethylacetamid |
| Emulsifier: | 1 part by weight of alkylaryl polyglycol ether |

To produce a suitable preparation of active compound, 1 part by weight of active compound or active compound combination is mixed with the stated amounts of solvent and emulsifier, and the concentrate is diluted with water to the desired concentration.

To test for preventive activity, young plants are sprayed with the preparation of active compound or active compound combination at the stated rate of application.

After the spray coating has dried on, the plants are sprayed with a spore suspension of ***Fusarium graminearum**.*

The plants are placed in a greenhouse under translucent incubation cloches at a temperature of approximately 22°C and a relative atmospheric humidity of approximately 100%.

The test is evaluated 5 days after the inoculation. 0% means an efficacy which corresponds to that of the control, while an efficacy of 100% means that no disease is observed.

In this test the following Compound 2 according to the invention showed an efficacy of 70% or even higher at a concentration of 1000ppm of active ingredient.

### Example F

### Production of Fumonisin FBI by Fusarium proliferatum

Compounds were tested in microtiter plates in 5 concentrations ranging from 0.08 µM to 50 µM in fumonisin-inducing liquid media (0.5g malt extract, 1g yeast extract, 1g bacto peptone, 20 g Fructose,1g KH₂PO₄, 0.3g MgSO₄x7H₂O, 0.3g KC1, 0.05g ZnSO₄x7H₂O and 0.01g CuSO₄x5H₂O per liter) containing 0.5% DMSO, inoculated with a concentrated spore suspension of *Fusarium proliferatum* to a final concentration of 2000 spores/ml.

Plates were covered and incubated at high humidity at 20 °C for 5 days

At start and after 5 days OD measurement at OD620 multiple read per well (square: 3 x 3) was taken to calculate growth inhibition.

After 5 days samples of each culture medium were taken and diluted 1:1000 in 50 % acetonitrile. The amounts of fumonisin FB1 of the samples were analysed per HPLC-MS/MS and results were used to calculate inhibition of FB1 production in comparison to a control without compound.

### Examples for inhibition of Fumonisin FB1 production

Compounds 1 and 2 showed an activity of > 80 % of inhibition of Fumonisin FB1 production at 50 µM. Growth inhibition of *Fusarium proliferatum* of these examples varied from 64 to 96 % at 50 µM.

### Example G

### Production of DON/Acetyl-DON by Fusarium graminearum

Compounds were tested in microtiter plates in 7 concentrations ranging from 0.07 µM to 50 µM in DON-inducing liquid media (1g (NH₄)₂HPO₄, 0.2g MgSO₄x7H₂O, 3g KH₂PO₄, 10g Glycerin, 5g NaCl and 40g Sachharose per liter), supplemented with 10 % oat extract, containing 0.5% DMSO, inoculated with a concentrated spore suspension of *Fusarium graminearum* to a final concentration of 2000 spores/ml.

The plate was covered and incubated at high humidity at 28°C for 7 days.

At start and after 3 days OD measurement at OD620 multiple read per well (square: 3 x 3) was taken to calculate the growth inhibition.

After 7 days 1 volume of 84/16 acetonitrile/water was added to each well and a sample of the liquid medium was taken and diluted 1:100 in 10 % acetonitrile. The amounts of DON and Acetyl-DON of the samples were analysed per HPLC-MS/MS and results were used to calculate inhibition of DON/AcDON production in comparison to a control without compound.

### Inhibition of DON/AcDON production

The compound 1 showed an activity of > 80 % of inhibition of DON/AcDON at 50 µM. Growth inhibition *of Fusarium graminearum* of this example was also > 80 % at 50 µM.

## Claims

1. A compound of formula (I) 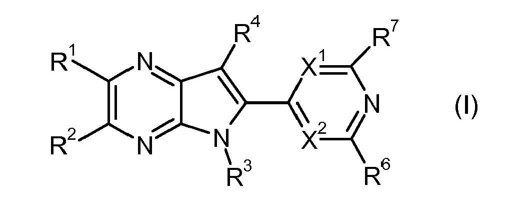 wherein:
X¹ is N or CH;
X₂ is N or CR⁵
R¹ and R² are, independently,:
(ix) hydrogen, halogen, hydroxyl, cyano or nitro,
(x) optionally substituted alkyl, alkenyl or alkynyl,
(xi) optionally substituted aryl, heteroaryl, cyclyl or heterocyclyl, or
(xii) C(O)R¹⁰, -C(O)NR¹⁰R¹¹, -C(S)NR¹⁰R¹¹, -C(NOR¹⁰)Rⁿ, -C(O)OR¹⁰, -OR¹⁰, -SR¹⁰,-S(O)R¹⁰, -S(O)NR¹⁰R¹¹, -S(O)₂NR¹⁰R¹¹, **-**S(O)₂R¹⁰**,** -NR¹⁰R¹¹,-NC(O)R¹⁰, -P(O)(OR¹⁰)(OR¹¹) or-OP(O)(OR¹⁰)(OR¹¹);
R³ is:
(i) hydrogen, hydroxyl, cyano or nitro,
(ii) optionally substituted alkyl, alkenyl, allenyl, alkynyl or haloalkyl, (iii) optionally substituted aryl, heteroaryl, cyclyl or heterocyclyl or heteroaralkyl or (iv) _C(O)R¹², -C(O)OR ¹², -OR¹², -OC(O)R¹², -S(O)₂R¹² or -NR¹²R¹³;
R⁴ is:
(ix) hydrogen, halogen, hydroxyl, cyano or nitro,
(x) (ii) optionally substituted alkyl, alkenyl, allenyl, alkynyl or haloalkyl,
(xi) (iii) optionally substituted aryl, heteroaryl, cyclyl or heterocyclyl or
(xii) -C(O)R¹⁴, -C(O)OR¹⁴, -C(NOR¹⁴)R¹⁵, -OR¹⁴, -SR¹⁴, -S(O)NR¹⁴R¹⁵, - S(O)₂R¹⁴, or -NR¹⁴R¹⁵;
R⁵ is:
(vii) hydrogen, halogen, hydroxyl, cyano or nitro,
(viii) optionally substituted alkyl, alkenyl or alkynyl,
(ix) -C(O)R¹⁶, -C(O)OR¹⁶, -OR¹⁶, -SR¹⁶, -S(O)R¹⁶, -S(O)NR¹⁶R¹⁷, -S(O)₂R¹⁶,
or -NR¹⁶R¹⁷;
R⁶ is hydrogen, halogen, cyano, -C(O)OR¹⁸, -SR¹⁸, -NR¹⁸R¹⁹, -C(O)NR¹⁸R¹⁹ or - N=CR²⁰, - C(=NR¹⁸)NR¹⁹R²⁰ or optionally substituted aryl, heteroaryl, cyclyl or heterocyclyl; ;
R⁷ is hydrogen, halogen, hydroxyl, cyano, nitro or optionally substituted alkyl;
R¹⁰, R¹¹, R¹⁴, R¹⁵, R¹⁶ and R¹⁷ are, independently, hydrogen, halogen, hydroxyl, cyano, nitro, optionally substituted alkyl, alkoxy, alkenyl or alkynyl, or optionally substituted aryl, heteroaryl, cyclyl or heterocyclyl;
R¹² and R¹³ are, independently, hydrogen, halogen, hydroxyl, cyano, nitro, -NR²¹ R²², optionally substituted alkyl, alkoxy, alkenyl or alkynyl, or optionally substituted aryl, heteroaryl, cyclyl or heterocyclyl;
R¹⁸ and R¹⁹ are, independently,
(ix) hydrogen,
(x) optionally substituted alkyl, alkenyl or alkynyl,
(xi) optionally substituted aryl, heteroaryl, cyclyl or heterocyclyl, or
(xii) -C(S)R²³ -C(O)R²³, -SO₂R²³ -C(O)OR²³, -OR²³ or C(O)NR²³R²⁴;
R²⁰ is hydroxyl, optionally substituted alkyl or alkoxy or -NR²¹R²², or -N=CR²¹R²²;
R²¹ and R²² are, independently, hydrogen, optionally substituted alkyl, alkenyl or alkynyl, optionally substituted cyclyl, heterocyclyl, aryl, or heteroaryl or aralkyl or -C(O)OR²⁵;
R²³ and R²⁴ are, independently, hydrogen, hydroxyl, optionally substituted alkyl, alkenyl or alkynyl, or optionally substituted aryl, heteroaryl, cyclyl or heterocyclyl or aralkyl; and
R²⁵ is optionally substituted alkyl, alkenyl or alkynyl;
and/or
independently, (i) R¹ and R², (ii) R³ and R⁵, (iii) R⁵ and R⁶ (iv) R⁵ and R¹⁸, (v) R⁵ and R¹⁹, (vi) R¹⁴ and R¹⁵ and (vii) R¹⁸ and R¹⁹ form an optionally substituted aryl, heteroaryl, cyclyl or heterocyclyl group containing from 5 to 18 ring atoms;
or a salt ofN-oxide thereof
with the proviso that the compounds from the group consisting of N-[4-[7-(4-fluorophenyl)-4H-pyrrolo[2,3-b]pyrazin-6-yl]-2-pyridinyl]-glycine, N1-[4-[7-(4-fluorophenyl)-5H-pyrrolo[2,3-b]pyrazin-6-yl]-2-pyridinyl]-1,2-ethanediamine, 4-[7-(4-fluorophenyl)-5H-pyrrolo[2,3-b]pyrazin-6-yl]-N-methyl-2-pyridinamine, 7-(4-fluorophenyl)-5-methyl-6-(4-pyridinyl)-5H-pyrrolo[2,3-b]pyrazine, 7-(3-chlorophenyl)-6-(2-chloro-4-pyridinyl)- 5H-pyrrolo[2,3-b]pyrazine, N-[4-[7-(4-fluorophenyl)-5H-pyrrolo[2,3-b]pyrazin-6-yl]-2-pyridinyl]-1-piperidineacetamide, 7-(4-fluorophenyl)-3-methoxy-6-(4-pyridinyl)- 5H-pyrrolo[2,3-b]pyrazine, 7-(4-fluorophenyl)-N,N-dimethyl-6-(4-pyridinyl)- 5H-pyrrolo[2,3-b]pyrazin-3-amine, 2-[[4-[7-(4-fluorophenyl)-3-(methylamino)-5H-pyrrolo[2,3-b]pyrazin-6-yl]-2-pyridinyl]amino]-ethanol, N-[4-[7-(4-fluorophenyl)-5H-pyrrolo[2,3-b]pyrazin-6-yl]-2-pyridinyl]-4-methyl-1-piperazine-acetamide, N-[4-[7-(4-fluorophenyl)-5H-pyrrolo[2,3-b]pyrazin-6-yl]-2-pyridinyl]- 1H-imidazole-1-acetamide, 7-(4-fluorophenyl)-N-methyl-6-[2-(methylamino)-4-pyridinyl]-5H-pyrrolo[2,3-b]pyrazin-3-amne, 4-[7-(3-chlorophenyl)-5H-pyrrolo[2,3-b]pyrazin-6-yl]-N-[2-(methylthio)ethyl]- 2-pyridinamine, 5-ethyl-7-(4-fluorophenyl)-6-(4-pyridinyl)- 5H-pyrrolo[2,3-b]pyrazine, N1-[4-[7-(4-fluorophenyl)-5H-pyrrolo[2,3-b]pyrazin-6-yl]-2-pyridinyl]- 1,3-propanediamine, 7-(4-fluorophenyl)-N-(3-methoxypropyl)-6-[2-[(3-methoxypropyl)amino]-4-pyridinyl]- 5H-pyrrolo[2,3-b]pyrazin-3-amine, 7-(4-fluorophenyl)-N-methyl-6-(4-pyridinyl)- 5H-pyrrolo[2,3-b]pyrazine-5-propanamine, N-[4-[3-(acetylamino)-7-(4-fluorophenyl)-5H-pyrrolo[2,3-b]pyrazin-6-yl]-2-pyridinyl]-acetamide, 3-chloro-6-(2-chloro-4-pyridinyl)-7-(4-fluorophenyl)- 5H-pyrrolo[2,3-b]pyrazine, 2-[[4-[7-(4-fluorophenyl)-5H-pyrrolo[2,3-b]pyrazin-6-yl]-2-pyridinyl] amino] -ethanol, N-[4-[3-[(3-aminopropyl)amino]-7-(4-fluorophenyl)-4H-pyrrolo[2,3-b]pyrazin-6-yl]-2-pyridinyl]- 1,3-propanediamine, 7-(4-fluorophenyl)-N-(2-methoxyethyl)-6-[2-[(2-methoxyethyl)amino]-4-pyridinyl]- 5H-pyrrolo[2,3-b]pyrazin-3-amine, 7-(4-fluorophenyl)-3-(methylthio)-6-(4-pyridinyl)- 5H-pyrrolo[2,3-b]pyrazine, 4-[[7-(4-fluorophenyl)-6-[2-[(4-hydroxybutyl)amino]-4-pyridinyl]-4H-pyrrolo[2,3-b]-pyrazin-3-yl]amino]- 1-butanol, N-[4-[7-(4-fluorophenyl)-5H-pyrrolo[2,3-b]pyrazin-6-yl]-2-pyridinyl]-2-methoxy-acetamide, 7-(4-fluorophenyl)-5-[3-(4-nitro-1H-imidazol-l-yl)propyl]-6-(4-pyridinyl)- 5H-pyrrolo[2,3-b]pyrazine, 2-[[7-(4-fluorophenyl)-6-[2-[(2-hydroxyethyl)amino]-4-pyridinyl]-4H-pyrrolo[2,3-b]pyrazin-3-yl]amino]- ethanol, 2-(dimethylamino)-N-[4-[7-(4-fluorophenyl)-5H-pyrrolo [2,3-b]pyrazin-6-yl]-2-pyridinyl]-acetamide, 3-[[7-(4-fluorophenyl)-6-[2-[[(2-hydroxyethyl)methyl]amino]-4-pyridinyl]-4H-pyrrolo[2,3-b]pyrazin-3-yl]ammo]-1-propanol, 7-(4-fluorophenyl)-5-[3-(2-nitro-1H-imidazol-1-yl)propyl]-6-(4-pyridinyl)- 5H-pyrrolo[2,3-b]pyrazine, 3-(dimethylamino)-N-[4-[7-(4-fluorophenyl)-5H-pyrrolo[2,3-b]pyrazin-6-yl]-2-pyridinyl]-propanamide, 7-(4-fluorophenyl)-5-[3-(4-methyl-1-piperazinyl)propyl]-6-(4-pyridinyl)-5H-pyrrolo[2,3-b]pyrazine, 7-(4-fluorophenyl)-5-[3-(4-morpholinyl)propyl]-6-(4-pyridinyl)- 5H-pyrrolo[2,3-b]pyrazine, N-[4-[7-(4-fluorophenyl)-5H-pyrrolo[2,3-b]pyrazin-6-yl]-2-pyridinyl]-2-(methylamino)-acetamide, N-[7-(4-fluorophenyl)-6-(4-pyridinyl)-5H-pyrrolo[2,3-b]pyrazin-3-yl]-acetamide, 6-(4-pyridinyl)- 5H-pyrrolo[2,3-b]pyrazine, 6-(2-bromo-4-pyridinyl)-7-(4-fluorophenyl)-5H-pyrrolo[2,3-b]pyrazine, 7-(4-fluorophenyl)-5-[3-(4-methyl-IH-imidazol-1-yl)propyl]-6-(4-pyridinyl)- 5H-pyrrolo[2,3-b]pyrazine, 5-(3-chloropropyl)-7-(4-fluorophenyl)-6-(4-pyridinyl)-5H-pyrrolo[2,3-b]pyrazine, 7-(4-fluorophenyl)-6-(4-pyridinyl)-5H-pyrrolo[2,3-b]pyrazine, N-[4-[7-(4-fluorophenyl)-5H-pyrrolo[2,3-b]pyrazin-6-yl]-2-pyridinyl]-acetamide, 4-[7-(4-fluorophenyl)-5H-pyrrolo[2,3-b]pyrazin-6-yl]-2-pyridinamine, 6-(2-chloro-4-pyridinyl)-7-(4-fluorophenyl)-5H-pyrrolo[2,3-b]pyrazine, 3-[[4-[7-(4-fluorophenyl)-5H-pyrrolo [2,3 -b]pyrazin-6-yl] -2-pyridinyl] ammo]-1-propanol, 7-(4-fluorophenyl)-5-[3-(IH-imidazol-1-yl)propyl]-6-(4-pyridinyl)-5H-pyrrolo[2,3-b]pyrazine, 7-(4-fluorophenyl)-6-(4-pyridinyl)-5H-pyrrolo[2,3-b]pyrazine-5-butanenitrile are excluded.

2. A compound of formula (I) according to claim 1 wherein
X¹ is CH.

3. A compound of formula (I) according to claim 1 or 2, wherein
X² is CH.

4. A compound of formula (I) according to any of the claims 1 to 3, wherein
R³ is hydrogen, -C(O)R¹², -C(O)OR¹², -OC(O)R¹², -S(O)₂R¹², optionally substituted C₁₋₆ alkyl, C₂₋₆ alkenyl, C₃₋₆ allenyl, C₂₋₆ alkynyl or optionally substituted saturated cyclyl.

5. A compound of formula (I) according to any of the claims 1 to 4,
wherein R⁴ is optionally substituted phenyl or thienyl.

6. A compound of formula (I) according to any of the claims 1 to 5,
wherein R⁶ is hydrogen or -NR¹⁸R¹⁹_{.}

7. A process for the preparation of a compound of general formula (I) as defined in any of the claim 1 to 6, which comprises the following steps as
in a first step (a) a compound of formula (II) 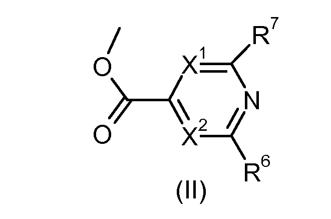 with R⁶ and R⁷ as defined above is reacted with a compound of formula (III) 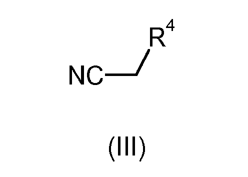 with R⁴ as defined above resulting in a compound of formula (IV) 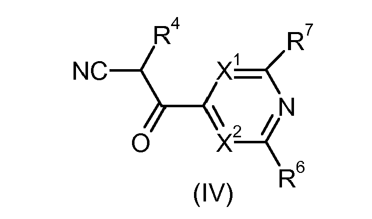 with R⁶, R⁷, and R⁴ as defined above,
the compound of formula (IV) is converted in a second step (b) to a ketone of formula (V) 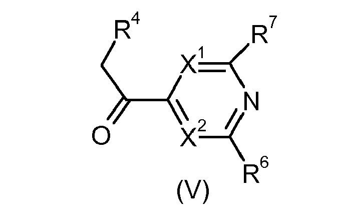 with R⁶, R⁷, and R⁴ as defined above,
the ketone of formula (V) is reacted in a third step (c) with a 2-hydrazinopyrazine of formula (VI) 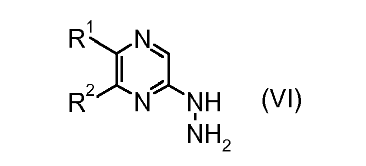 with R¹ and R² as defined above in the presence of a catalytic amount of an acid to a hydrazone of formula (VII) 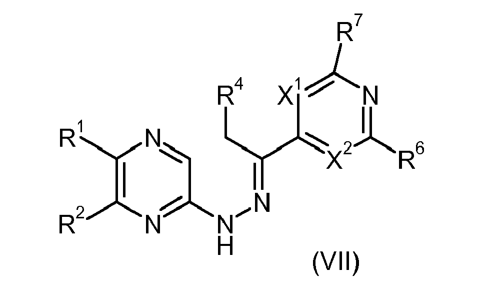 with R¹, R², R⁶, R⁷, and R⁴ as defined above,
the hydrazone of formula (VII) is converted in a fourth step (d) to a 5H-pyrrolo[2,3-b]pyrazine of formula (Ie) 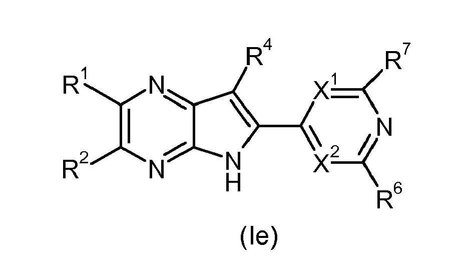 with R¹, R², R⁶, R⁷, and R⁴ as defined above,
by heating the hydrazone of formula (VII) in a high boiling solvent such as diethylene glycol to its boiling point,
in a fifth step (e) the compound according to formula (Ie) is converted to a compound according to formula (I) in an aprotic solvent in the presence of a strong base.

8. A composition for preventing and/or controlling fungal infection in plants and/or plant propagation material and for reducing mycotoxin contamination in plants or plant material, **characterized in that** they comprise at least one compound of the formula (I) according to any of the claims 1 to 6 or wherein the substituents within formula (I) are defined as:
X¹ is N or CH;
X₂ is N or CR⁵
R¹ and R² are independently
(xiii) hydrogen, halogen, hydroxyl, cyano or nitro,
(xiv) optionally substituted alkyl, alkenyl or alkynyl,
(xv) optionally substituted aryl, heteroaryl, cyclyl or heterocyclyl, or
(xvi) C(O)R¹⁰, -C(O)NR¹⁰R¹¹, -C(S)NR¹⁰R¹¹, -C(NOR¹⁰)Rⁿ, -C(O)OR¹⁰, -OR¹⁰,-SR¹⁰,-S(O)R¹⁰, -S(O)NR¹⁰R¹¹, -S(O)₂NR¹⁰R¹¹, -S(O)₂R¹⁰, -NR¹⁰R¹¹, -NC(O)R¹⁰ -P(O)(OR¹⁰)(OR¹¹) or-OP(O)(OR¹⁰)(OR¹¹);
R³ is
(i) hydrogen, hydroxyl, cyano or nitro,
(ii) optionally substituted alkyl, alkenyl, allenyl, alkynyl or haloalkyl, (iii) optionally substituted aryl, heteroaryl, cyclyl or heterocyclyl or heteroaralkyl or (iv) -C(O)R¹², -C(O)OR¹², -OR¹², -OC(O)R¹², -S(O)₂R¹² or -NR¹²R¹³;
R⁴ is
(xiii) hydrogen, halogen, hydroxyl, cyano or nitro,
(xiv) optionally substituted alkyl, alkenyl, allenyl, alkynyl or haloalkyl,
(xv) optionally substituted aryl, heteroaryl, cyclyl or heterocyclyl or
(xvi) -C(O)R¹⁴ , -C(O)OR¹⁴, -C(NOR¹⁴)R¹⁵, -OR¹⁴, -SR¹⁴, -S(O)NR¹⁴R¹⁵, - S(O)₂R¹⁴, or -NR¹⁴R¹⁵;
R⁵ is
(x) hydrogen, halogen, hydroxyl, cyano or nitro,
(xi) optionally substituted alkyl, alkenyl or alkynyl,
(xii) -C(O)R¹⁶, -C(O)OR¹⁶, -OR¹⁶, -SR¹⁶, -S(O)R¹⁶, -S(O)NR¹⁶R¹⁷, -S(O)₂R¹⁶, or -NR¹⁶R¹⁷;
R⁶ is hydrogen, halogen, cyano, -C(O)OR¹⁸, -SR¹⁸, -NR¹⁸R¹⁹, -C(O)NR¹⁸R¹⁹ or - N=CR²⁰, - C(=NR¹⁸)NR¹⁹R²⁰ or optionally substituted aryl, heteroaryl, cyclyl or heterocyclyl;
R⁷ is hydrogen, halogen, hydroxyl, cyano, nitro or optionally substituted alkyl;
R¹⁰, R¹¹, R¹⁴, R¹⁵, R¹⁶ and R¹⁷ are, independently, hydrogen, halogen, hydroxyl, cyano, nitro, optionally substituted alkyl, alkoxy, alkenyl or alkynyl, or optionally substituted aryl, heteroaryl, cyclyl or heterocyclyl;
R¹² and R¹³ are, independently, hydrogen, halogen, hydroxyl, cyano, nitro, -NR²¹ R²², optionally substituted alkyl, alkoxy, alkenyl or alkynyl, or optionally substituted aryl, heteroaryl, cyclyl or heterocyclyl;
R¹⁸ and R¹⁹ are, independently,
(xiii) hydrogen,
(xiv) optionally substituted alkyl, alkenyl or alkynyl,
(xv) optionally substituted aryl, heteroaryl, cyclyl or heterocyclyl, or
(xvi) -C(S)R²³-C(O)R²³, -SO₂R²³, -C(O)OR²³, -OR²³ or C(O)NR²³R²⁴;
R²⁰ is hydroxyl, optionally substituted alkyl or alkoxy or -NR²¹R²², or -N=CR²¹R²²;
R²¹ and R²² are, independently, hydrogen, optionally substituted alkyl, alkenyl or alkynyl, optionally substituted cyclyl, heterocyclyl, aryl, or heteroaryl or aralkyl or -C(O)OR²⁵;
R²³ and R²⁴ are, independently, hydrogen, hydroxyl, optionally substituted alkyl, alkenyl or alkynyl, or optionally substituted aryl, heteroaryl, cyclyl or heterocyclyl or aralkyl; and
R²⁵ is optionally substituted alkyl, alkenyl or alkynyl;
and/or
independently, (i) R¹ and R² (ii) R³ and R⁵, (iii) R⁵ and R⁶ (iv) R⁵ and R¹⁸, (v) R⁵ and R¹⁹, (vi) R¹⁴ and R¹⁵ and (vii) R¹⁸ and R¹⁹ form an optionally substituted aryl, heteroaryl, cyclyl or heterocyclyl group containing from 5 to 18 ring atoms;
or a salt or a N-oxide thereof, in addition to extenders and/or surfactants.

9. A composition for reducing mycotoxin contamination in plants or plant material, **characterized in that** they comprise at least one compound of the formula (I) according to any of the claims 1 to 6 and 8, in addition to extenders and/or surfactants.

10. Use of compounds of formula (I) according to any of the claims 1 to 6 and 8 for preventing and/or controlling fungal infection in plants and/or plant propagation material and for reducing mycotoxin contamination in plants or plant material.

11. Use of compounds of formula (I) according to any of the claims 1 to 6 and 8 for reducing mycotoxin contamination in plants or plant material.

12. Method for preventing and/or controlling fungal infection in plants and/or plant propagation material and for reducing mycotoxin contamination in plants or plant material **characterized in that** compounds of the formula (I) according to any of the claims 1 to 6 and 8 are applied to the fungi and/or their habitat.

13. Method for reducing mycotoxin contamination in plants or plant material **characterized in that** compounds of the formula (I) according to any of the claims 1 to 6 and 8 are applied to the fungi and/or their habitat.

14. Process for preparing compositions for preventing and/or controlling fungal infection in plants and/or plant propagation material and for reducing mycotoxin contamination in plants or plant material, **characterized in that** compounds of the formula (I) according to any of the claims 1 to 6 and 8 are mixed with extenders and/or surfactants.

15. Use of compounds of formula (I) according to any of the claims 1 to 6 and 8 for preventing and/or controlling fungal infection in transgenic plants and/or transgenic plant propagation material and for reducing mycotoxin contamination in transgenic plants or transgenic plant material
